(19) Europäisches Patentamt
European Patent Office
Office européen des brevets

(11) EP 3 059 005 B1

(12) **EUROPÄISCHE PATENTSCHRIFT**

(45) Veröffentlichungstag und Bekanntmachung des Hinweises auf die Patenterteilung:
**24.10.2018 Patentblatt 2018/43**

(51) Int Cl.:
***B01D 61/02*** *(2006.01)* ***B01D 61/12*** *(2006.01)*
***B01J 31/40*** *(2006.01)* ***C07C 45/50*** *(2006.01)*

(21) Anmeldenummer: **16155014.0**

(22) Anmeldetag: **10.02.2016**

(54) **ABTRENNUNG EINES HOMOGENKATALYSATORS AUS EINEM REAKTIONSGEMISCH MIT HILFE ORGANOPHILER NANOFILTRATION UNTER BESONDERER BERÜCKSICHTIGUNG EINES MEMBRAN-LEISTUNGSINDIKATORS**

SEPARATION OF A HOMOGENEOUS CATALYST FROM A REACTION MIXTURE USING ORGANOPHILIC NANOFILTRATION UNDER CONSIDERATION OF A MEMBRANE PERFORMANCE INDICATOR

SEPARATION D'UN CATALYSEUR HOMOGENE D'UN MELANGE REACTIF A L'AIDE D'UNE NANOFILTRATION ORGANOPHILIQUE EN TENANT COMPTE NOTAMMENT D'UN INDICATEUR DE PERFORMANCE A MEMBRANE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.02.2015 EP 15155497**

(43) Veröffentlichungstag der Anmeldung:
**24.08.2016 Patentblatt 2016/34**

(73) Patentinhaber: **Evonik Degussa GmbH**
**45128 Essen (DE)**

(72) Erfinder:
- **Geilen, Frank**
  **45721 Haltern am See (DE)**
- **Franke, Robert**
  **45772 Marl (DE)**
- **Hamers, Bart**
  **5961 RM Horst (NL)**

(56) Entgegenhaltungen:
**WO-A1-2015/014741 DE-A1-102012 223 572**
**DE-A1-102013 208 759**

- **PRISKE M ET AL: "Reaction integrated separation of homogenous catalysts in the hydroformylation of higher olefins by means of organophilic nanofiltration", JOURNAL OF MEMBRANE SCIENCE, ELSEVIER, Bd. 360, Nr. 1-2, 15. September 2010 (2010-09-15), Seiten 77-83, XP027118372, ISSN: 0376-7388 [gefunden am 2010-07-02]**
- **MARRIOTT J ET AL: "The optimal design of membrane systems", CHEMICAL ENGINEERING SCIENCE, OXFORD, GB, Bd. 58, Nr. 22, 1. November 2003 (2003-11-01), Seiten 4991-5004, XP004468452, ISSN: 0009-2509, DOI: 10.1016/J.CES.2003.07.011**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 059 005 B1

## Beschreibung

Die Erfindung befasst sich mit der Abtrennung von Homogenkatalysatoren aus Reaktionsgemischen mit Hilfe der organophilen Nanofiltration.

Die Membrantechnik ist eine vergleichsweise junge Technologie zum Trennen von Stoffgemischen. Ihr Grundprinzip besteht darin, das zu trennende Stoffgemisch auf eine Membran zu geben, die für die einzelnen im Gemisch enthaltenen Komponenten eine unterschiedliche Durchlässigkeit aufweist. Dies führt dazu, dass die in dem zu trennenden Stoffgemisch enthaltenden Komponenten unterschiedlich schnell durch die Membran hindurchtreten (permeieren) und sich dementsprechend beiderseits der Membran unterschiedlich stark konzentrieren. Trennkriterium ist mithin die Durchlässigkeit der Membran hinsichtlich des abzutrennenden Stoffes. Treibende Kraft ist meist ein Druckgefälle zwischen beiden Seiten der Membran, der sogenannte Transmembrandruck $\Delta p$. Daneben werden auch andere Triebkräfte genutzt.

Die Membrantechnik nutzt dabei nicht nur den mechanischen Siebeffekt, der Komponenten nach unterschiedlichen Partikelgrößen selektiert, sondern auch Lösungs- und Diffusionseffekte. Eine Membran wirkt damit deutlich komplexer als ein einfacher mechanischer Filter und kann deswegen auch Flüssigkeiten und/oder Gase voneinander trennen.

Im konkreten technischen Aufbau wird das zu trennende Gemisch als Feed auf die Membran gefahren. Dort wird es getrennt in ein Retentat diesseits der Membran und in ein Permeat jenseits der Membran. Permeat und Retentat werden kontinuierlich von der Membran abgezogen. Aufgrund der Trennwirkung reichern sich im Permeat die Komponenten an, für die die Membran eine hohe Durchlässigkeit aufweist, währenddessen sich im Retentat die Stoffe ansammeln, für welche die Membran weniger durchlässig ist. Da viele Membranprozesse Membranen einsetzen, die grundsätzlich für alle Komponenten des Stoffgemisches durchlässig sind - eben nur mit unterschiedlichen Durchtrittsgeschwindigkeiten - trennen solche Membranprozesse nicht digital sondern es finden sich durchaus alle Komponenten des Stoffgemisches sowohl im Retentat als auch im Permeat, jedoch in unterschiedlicher Konzentration (Massenanteil).

Deswegen wird in der Membrantechnik zur Charakterisierung der Durchlässigkeit einer Membran der Rückhalt R der Membran hinsichtlich einer bestimmten Komponente des Stoffgemisches wie folgt definiert:

$$R := 1 - w_P/w_R$$

worin wpfürden Massenanteil der betrachteten Komponente im Permeat und $w_R$ für den Massenanteil der betrachteten Komponente im Retentat der Membran steht. Der Rückhalt kann somit Werte von 0 bis 1 annehmen und wird deswegen gerne in % angegeben. Betrachtet man ein einfaches 2-Komponenten-System, so bedeutet zum Beispiel ein Rückhalt von 0 %, dass die betrachtete Komponente genau gleichgut wie das Lösungsmittel permeiert, sodass die Massenanteile der Komponenten im Retentat wie im Permeat gleich sind. Andererseits bedeutet ein Rückhalt von 100, dass die betrachtete Komponente vollständig zurückgehalten wird. Neben dem Rückhalt bezüglich der abzutrennenden Komponente ist auch die sogenannte Permeabilität der Membran für die Charakterisierung ihrer Durchlässigkeit maßgeblich:

$$P : = m' / (A * \Delta p)$$

Worin m' für den Massenstrom des Permeats, A für die Fläche der Membran und $\Delta p$ für den angelegten Transmembrandruck steht. Angegeben wird die Permeabilität in der Regel in der Einheit kg /(h * $m^2$ * bar).

Permeabilität P und Rückhalt R sind vorgegeben von dem trennaktiven Material der Membran sowie von der Zusammensetzung des zu trennenden Stoffgemisches. Bei der Auslegung eines Membrantrennprozesses sind diese Parameter stets beachtlich. Aufgrund der Stoffabhängigkeit dieser Parameter werden sie durch die Wahl des Membranmaterials festgelegt. Deswegen ist Auswahl des trennaktiven Membranmaterials für die jeweilige Trennaufgabe maßgeblich für die gesamte Prozessauslegung.

Die bis hierhin wiedergegebenen und weitere Grundlagen der Membrantechnologie können nachgelesen werden bei Melin / Rautenbach: Membranverfahren. Grundlagen der Modul- und Anlagenauslegung. Springer, Berlin Heidelberg 2004.

Abhängig von der Größe der abzutrennenden Spezies, der genutzten Trenneffekte und der Triebkraft unterscheidet man unterschiedliche Klassen der Membranen bzw. der Membrantrennverfahren. Gängig sind die Klassen Ultrafiltration, Nanofiltration, Gaspermeation und Umkehrosmose. Diese Begrifflichkeiten werden nicht einheitlich verwendet und sind nicht scharf voneinander abgegrenzt. Sofern hier von einer Nanofiltration die Rede ist, ist die Abtrennung von Molekülen mit einer Molmasse von über 300 g/mol mit Hilfe einer Membran aus einem zumindest teilweise flüssigen Stoffgemisch gemeint. Da solche Moleküle einen Durchmesser in der Größenordnung von 1 nm aufweisen, spricht man von Nanofiltration bzw. einer Nanofiltrationsmembran.

Je nachdem, ob ein überwiegend wässriges Stoffgemisch getrennt wird oder ein überwiegend organisches

Stoffgemisch, spricht man von wässriger Nanofiltration oder von organophiler Nanofiltration. Da die Beständigkeit der Membranmaterialien und insbesondere ihr Quellverhalten im wässrigen und organischen Milieu sehr unterschiedlich ausfallen, ist diese Unterscheidung für den Membrantechniker relevant.

Ein Einsatzgebiet, in dem sich die organophile Nanofiltration einsetzen lässt, ist die Abtrennung von Katalysatoren bzw. deren Bestandteilen oder Abbauprodukten aus Reaktionsgemischen, die aus homogen katalysierten chemischen Reaktionen stammen.

Katalysatoren dienen dazu chemische Reaktionen zu beschleunigen ohne dabei selbst verbraucht zu werden. In der industriellen Chemie machen Katalysatoren die Durchführung von Reaktionen erst wirtschaftlich oder überhaupt erst möglich. Deswegen sind Katalysatoren in der chemischen Industrie von großem Wert. Da die Katalysatoren nicht verbraucht werden, können sie nach Abschluss der mit ihnen katalysierten Reaktion abgetrennt und wiederverwertet werden. Der Aufwand, der für Katalysatorabtrennung betrieben werden muss bzw. betrieben wird, hängt von dem Wert und der Art des Katalysators ab.

Unterschieden werden zwei Arten von Katalysatoren, nämlich homogene und heterogene. Homogene Katalysatoren liegen in derselben Phase des Reaktionsgemisches wie die Reaktionsteilnehmer vor, währenddessen sich heterogene Katalysatoren in einer anderen Phase befinden. Bei den heterogenen Katalysatoren handelt es sich meist um Festkörper, die leicht von dem flüssigen oder gasförmigen Reaktionsgemisch abtrennen lassen. Günstigstenfalls bedarf es dafür noch nicht mal eines Siebs oder Filters, da der feste Katalysator einfach im Reaktor verbleibt, währenddessen das flüssige Reaktionsgemisch abgezogen wird. Aufwendig ist indes die Abtrennung von Homogenkatalysatoren, die in dem flüssigen Reaktionsgemisch gelöst sind. Dies ist aufgrund des molekularen Zustands des gelösten Homogenkatalysators mit einem Filter nicht möglich. Deswegen ist eine Destillation, Extraktion oder eben eine Membrantrennung erforderlich. Da Homogenkatalysatoren Teilchengrößen zwischen 0.5 und 3 nm erreichen, ist zu deren Abtrennung eine Nanofiltrationsmembran erforderlich.

Ein Gebiet der industriellen Chemie, auf dem die organophile Nanofiltration erfolgreich eingesetzt werden kann, um wertvollen Homogenkatalysator aus katalysierten flüssigen Reaktionsgemischen abzutrennen ist die Hydroformylierung von Olefinen (Alkenen). Bei dieser auch Oxo-Reaktion genannten Synthese werden Olefine mit n Kohlenstoffatomen mit einer Mischung aus Wasserstoff und Kohlenmonoxid - dem sogenannten Synthesegas - zu Aldehyden mit n+1 Kohlenstoffatomen umgesetzt. Die Aldehyde werden meist durch Hydrierung in Alkohole überführt, die aufgrund ihrer Genese auch Oxo-Alkohole genannt werden.

Grundsätzlich sind alle Olefine der Hydroformylierung zugänglich, in der Praxis werden jedoch meist solche Olefine als Substrat in der Hydroformylierung eingesetzt, die zwei bis 20 Kohlenstoffatome aufweisen. Da die durch Hydroformylierung und Hydrierung erhältlichen Alkohole vielfältig eingesetzt werden können - etwa als Weichmacher für PVC, als Detergentien in Waschmitteln und als Riechstoffe - wird die Hydroformylierung großindustriell praktiziert.

Als Katalysator werden in der Hydroformylierung unter anderem metallorganische Komplexverbindungen genutzt, die ein Metall als Zentralatom aufweisen, welches mit verschiedenen Liganden komplexiert ist. Häufig werden als Liganden Organophosphor-Verbindungen eingesetzt, nicht limitierende Beispiele sind Organophosphine,Organophosphite oder Organophosphoramidite. Ein solches Katalysatorsystem wird in dem flüssigen Reaktionsgemisch aus Olefin und darin gelösten Synthesegas gelöst und ist damit homogen. Die Abtrennung erfolgt aus dem abgezogenen Reaktionsgemisch enthaltend die gebildeten Aldehyde, Nebenprodukte, nicht umgesetzte Edukte und eben gelöster Homogenkatalysator oder Bestandteile oder Abbauprodukte desselben, also reines Metall, freie Liganden oder degeneriertes Metall und Liganden, wobei man unter degeneriertem Metall beispielsweise mehrkernige MetallCarbonyl-Cluster verstehen kann, die ggf. noch Ligandmodifiziert sind. Industriell kommen Katalysatorsysteme zum Einsatz, die Cobalt oder Rhodium als Zentralatom aufweisen, wobei letztere oft mit Organo-Phosphor-Liganden wie Phosphin-, Phosphit- oder Phosphoramidit-Verbindungen komplexiert werden.

Insbesondere die Abtrennung von Rh-basierten Katalysatorkomplexen aus homogen katalysierten Hydroformylierungsmischungen erweist sich als technisch anspruchsvoll. Dies liegt zum einen daran, dass Rh ein sehr teures Edelmetall ist, dessen Verlust es tunlichst zu vermeiden gilt. Aus diesem Grunde muss das Rhodium aus dem Produktstrom möglichst vollständig abgetrennt und zurückgewonnen werden. Da die Rh-Konzentration in typischen Hydroformylierungsreaktionen lediglich 5 bis 200 ppm beträgt und eine typische "world scale" Oxo-Anlage eine Jahresleistung von 200 000 Tonnen oder mehr erzielt, müssen Trennapparate eingesetzt werden, die einerseits einen großen Durchsatz erlauben und andererseits das nur in geringen Mengen enthaltene Rh sicher abtrennen. Erschwerend kommt hinzu, dass die zum Katalysator-Komplex gehörigen Organo-Phosphor-Liganden sehr empfindlich auf Zustandsänderungen reagieren und rasch degenerieren. Da die Zersetzungsprodukte der Liganden entweder die metallorganischen Komplexe nicht mehr zu stabilisieren vermögen oder aber besonders stark an die Metallzentren binden, wird das empfindliche, für die erfolgreiche katalytische Reaktion notwendige Gleichgewicht am Katalysatorkomplex gestört. Dies führt makroskopisch zu einer Desaktivierung des Katalysators. Ein desaktivierter Katalysator ist bestenfalls nur aufwändig zu reaktivieren. Die Katalysatorabtrennung muss deshalb besonders schonend erfolgen. Ein weiteres wichtiges Entwicklungsziel sind die Investitionskosten der Katalysatorabtrennung, sprich der NanofiltrationsAnlage.

Über die Möglichkeiten des Einsatzes der Membrantechnologie zur Aufarbeitung von Hydroformylierungsgemischen berichten Priske, M. et al.: Reaction integrated separation of homogeneous catalysts in the hydroformylation of higher olefins by means of organophilic nanofiltration. Journal of Membrane Science, Volume 360, Issues 1-2, 15 September 2010, Pages 77-83; doi:10.1016/j.memsci.2010.05.002.

Einen weitergehenden Überblick über verschiedene lösemittelbasierende Nanofiltrations-Prozesse (Organic Solvent Nanofiltration, OSN) gibt die Literaturstelle: Chem. Soc. Rev., 2008, 37, 365-405. Ein großer Vorteil der Membrantrennverfahren im Vergleich zu thermischen Trennverfahren ist der geringere Energieeinsatz; allerdings stellt sich auch bei Membrantrennverfahren das Problem der Desaktivierung des Katalysatorkomplexes.

Gelöst wurde dieses Problem durch das in EP1931472B1 beschriebene Verfahren zur Aufarbeitung von Hydroformylierungsgemischen, bei dem sowohl im Feed, im Permeat und auch im Retentat der Membran ein bestimmter Kohlenmonoxid-Partialdampfdruck aufrechterhalten wird. Dadurch gelingt es erstmals, die Membrantechnologie wirksam in der industriellen Hydroformylierung einzusetzen.

Ein weiteres Beispiel in der Patentliteratur, welches sich mit der Membranabtrennung von Homogenkatalysatoren aus Hydroformylierungsgemischen beschäftigt, ist WO2014/183952A1. Dort wird der Rückhalt einer Membrantrenneinrichtung aktiv geregelt, um so den Retentatvolumenstrom zu verstetigen und damit letztendlich Störungen der Hydrodynamik des Reaktors entgegenzuwirken.

Ein spezifischer Nachteil von Membrantrennverfahren ist, dass diese noch vergleichsweise junge Technologie mit der Verfügbarkeit der Membranen steht und fällt. Insbesondere die trennaktiven Materialien der Membran, welche letztendlich die Abtrennung der Katalysatorkomplexe bewerkstelligen und somit über die Erfüllung der Trennaufgabe entscheiden, sind noch nicht in großen Mengen oder zu geringen Preisen verfügbar. Die Auftrennung großer Stoffströme erfordert aber sehr große Membranflächen und damit verbunden entsprechend viel Material und hohe Investitionskosten. Dies führt dazu, dass sich die technisch inzwischen reife Technologie nicht überall wirtschaftlich einsetzen lässt. Dieses Problem konnte bisher nicht verfahrenstechnisch gelöst werden. Es betrifft nicht nur die Hydroformylierung, sondern auch andere homogen katalysierte Reaktionen, die im industriellen Maßstab durchgeführt werden.

Die Optimierung von Membrantrennprozessen unter wirtschaftlichen Randbedingungen wird im Stand der Technik untersucht, siehe z.B. Marriot J et al, "the optimal design of membrane systems", Chm. Eng. Sc, Oxford. GB, Bd. 58, Nr. 22, S. 4991-5004 (2003)

Aufgabe ist es daher, eine Möglichkeit aufzuzeigen, wie sich die organophile Nanofiltration zur Abtrennung von Homogenkatalysatoren aus Reaktionsgemischen wirtschaftlich einsetzen lässt, wenn das die Trennaufgabe erfüllende, trennaktive Material der Membran nicht in ausreichend großen Mengen oder nur zu hohen Kosten verfügbar ist.

Gelöst wird diese Aufgabe durch die Berücksichtigung eines besonderen Membran-Leistungsindikators bei der Ausübung des entsprechenden Membrantrennverfahrens nach Anspruch 1 bzw. durch die Vorrichtung nach Anspruch 2..

Der gemäß der Erfindung verwendete Membran-Leistungsindikator MPI ist wie folgt definiert:

$$\mathrm{MPI} := 0.5\ R^{S/7}\ (1+P)^{S/(100*R)} - 1/(10*P)$$

worin R für den dimensionslosen Rückhalt der Membran hinsichtlich eines Metalls und P für die Permeabilität der Membran in kg/(m$^2$ *bar*h) steht und worin S für einen angenommenen bzw. den gegenwärtigen Marktpreis des Metalls in Euro/g steht.

Ein wirtschaftlicher Betrieb des Membrantrennverfahrens ist dann gewährleistet, wenn der Membran-Leistungsindikator MPI mindestens 0.35 beträgt. Besser noch beträgt der MPI mindestens 0.5 und optimal mehr als 0.75. Je höher der MPI, desto besser die Wirtschaftlichkeit. Mit dem Membran-Leistungsindikator lassen sich deswegen Membranprozesse hinsichtlich ihrer Wirtschaftlichkeit vergleichen und auslegen. Wenngleich der Membran-Leistungsindikator MPI Werte mathematisch größer 1 und kleiner 0 annehmen kann, ist seine Verwendung nur zwischen 0 und 1 sinnvoll. Bisher sind nämlich keine Membranen bekannt, die einen MPI größer 1 erreichen. Der MPI wird daher begrenzt betrachtet für Werte zwischen 0 und 1. Negative Werte werden als Null angesetzt, Werte größer 1 auf 1. Zwischen 0 und 1 werden die Werte auf Vielfache von 0,05 gerundet, wobei Werte von x,00 bis x,024 sowie von x,05 bis x,074 abgerundet und Werte von x,025 bis x,049 sowie von x,075 bis x,099 aufgerundet werden.

Die Besonderheit des hier definierten Membran-Leistungsindikators MPI besteht darin, dass er gar nicht auf die eigentlichen Kosten des Membranmaterials Bezug nimmt, sondern sich vielmehr an den Katalysatorkosten orientiert: Der Parameter Marktpreis S des Metalls bezieht sich nämlich erfindungsgemäß auf das Metall, welches als Zentralatom in der homogenkatalytisch aktiven metallorganischen Komplexverbindung eingesetzt wird.

Dahinter steht der Grundgedanke, den Marktpreis des Metalls als Maß für die Abtrennungsgüte zu verwenden: So kann es wirtschaftlich durchaus Sinn machen, die Nanofiltration deutlich schlechter zu fahren als technisch möglich und dabei vergleichsweise viel Katalysator zu verlieren, sofern der Katalysator preisgünstig ist. Umgekehrt macht es keinen Sinn viel Geld in große Mengen teures Membranmaterial zu stecken um damit viel preisgünstiges Metall zurückzugewinnen.

Die Eigenschaft, ob eine Membran nun eher durchlässig oder eher undurchlässig für das abzutrennende Metall ist, wird durch die beiden anderen Parameter des Membran-Leistungsparameters, nämlich der Rückhalt der Membran hinsichtlich des zur Rede stehenden Metalls und ihrer sich aus der Prozessführung ergebenden Permeabilität, bestimmt. Auf diese Weise verknüpft der gefundene Membran-Leistungsparameter Material- und Prozessparameter mit dem extern vorgegebenen Preis.

Bei einigen als Homogenkatalysator eingesetzten metallorganischen Komplexen ist das als Zentralatom fungierende Metall nicht der Kostentreiber, sondern die organischen Liganden, welche sich an das Metallatom komplexieren. In solchen Konstellationen macht es Sinn, den Marktpreis S nicht auf das Metall zu beziehen, sondern auf den tatsächlich Kostenrelevanten Bestandteil des Katalysatorsystems, etwa der Ligand oder dessen Abbauprodukte. Der Rückhalt R ist dann ebenfalls hinsichtlich dieses kostenrelevanten Bestandteils des Katalysatorsystems und/oder dessen Abbauprodukte zu beziehen. Allgemein kann mit dem Verfahren anstelle des (katalytisch aktiven) Metalls auch ein anderer kostenrelevanter Wertstoff abgeschieden werden. Marktpreis und Rückhalt sind dann entsprechend auf die abzuscheidenden Wertstoff zu beziehen.

Die Anwendung des Membran-Leistungsparameters führt zu überraschenden Erkenntnissen dahingehend, dass eine durchlässige Membran durchaus einer dichten Membran vorzuziehen ist. In der Vergangenheit galt es vielmehr die Membran zu nutzen, die am wenigsten Katalysator durchlässt.

Eine Besonderheit des Membran-Leistungsparameters MPI besteht auch darin, dass er nicht eine bloße wirtschaftliche Kenngröße darstellt, sondern sich unmittelbar technisch nutzen lässt, nämlich einerseits bereits in der Planungsphase zur Auslegung des Membrantrennverfahrens und andererseits während des laufenden Betriebs des Membrantrennverfahrens, genauer gesagt, zur Regelung der eingesetzten Nanofiltration.

Aufgrund der vielfältigen Einsatzmöglichkeiten des Membran-Leistungsindikators ist eine Vielzahl von Verfahren und Vorrichtungen auf dem Gebiet der Abtrennung von Homogenkatalysatoren mit Hilfe der organophiler Nanofiltration Gegenstand der Erfindung, deren gemeinsames Merkmal die Berücksichtigung dieses Membran-Leistungsindikators ist.

Eine erste Möglichkeit, besteht darin, den Membran-Leistungsindikator bereits in der Planungsphase der Nanofiltration zu nutzen, um das Abtrennungsverfahren richtig zu dimensionieren.

Diese Möglichkeit besteht mithin in einem Verfahren zur Auslegung eines Verfahrens zur Abtrennung eines Metalls aus einem Reaktionsgemisch mittels einer Membran, bei welchem das Verfahren zur Abtrennung modelliert und dabei der dimensionslose Rückhalt R der Membran hinsichtlich des Metalls (bzw. des kostenrelevanten Bestandteils und/oder dessen Abbauprodukte) und die Permeabilität P der Membran in kg/(m2*bar*h) bestimmt wird, wobei unter Annahme eines Marktpreises S des Metalls (bzw. des
kostenrelevanten Bestandteils und/oder dessen Abbauprodukte) in Euro/g ein dimensionsloser Membran-Leistungsindikator MPI wie folgt bestimmt wird:

$$MPI := 0.5\ R^{S/7}\ (1+P)^{S/(100*R)} - 1/(10*P)$$

und wobei zumindest einer der Parameter Rückhalt, Permeabilität und Marktpreis solange variiert und nach Variation des Parameters der Membran-Leistungsindikator erneut bestimmt wird, bis der erneut bestimmte Membran-Leistungsindikator mindestens 0.35 beträgt.

Die Vorgehensweise besteht also darin, die Nanofiltration zunächst mathematisch zu simulieren und dabei grob die Verfahrensparameter der späteren Anlage voreinzustellen. Zumindest die Stoffströme durch die Nanofiltrationsanlage sind anzunehmen. Darüber hinaus wird ein trennaktives Membranmaterial gewählt, welches die Trennaufgabe grundsätzlich erfüllt. Für dieses Material sind die grundlegenden Kenngrößen Permeabilität und Rückhalt und ggf. deren Abhängigkeiten von den Prozessparametern Transmembrandruck und Temperatur einmalig experimentell unter relevanten Prozessbedingungen zu bestimmen. Jedes trennaktive Membranmaterial hat für ein zu trennendes Stoffsystem und bei standardisierten Rahmenbedingungen ein charakteristisches Verhältnis von Rückhalt und Permeabilität. Durch Variation von Prozessparametern, wie Transmembrandruck und Temperatur kann aber innerhalb eines begrenzten Parameterfensters Einfluss auf den Rückhalt und den absoluten Massenfluss durch die Membran genommen werden. Die Einflüsse sollten im Bedarfsfall ebenfalls einmalig bestimmt werden. Nun wird ein Markpreis des Metalls angenommen, welches das Zentralatom des abzutrennenden Katalysators darstellen wird. Bei der Annahme des Marktpreises muss natürlich eingeschätzt werden, wie sich der Preis des Metalls in der Zukunft entwickelt. Hierzu können zum Beispiel Wahrscheinlichkeiten auf Basis einer Monte-Carlo-Simulation aus historischen Daten bestimmt werden. Sodann kann der MPI nach vorstehender Formel berechnet werden. Wenn der errechnete MPI unter 0.35 liegt und die Druck und Temperaturabhängigkeiten bekannt sind, können die Betriebsparameter Temperatur und Druck der Nanofiltration in engen Grenzen verändert werden, sodass sich Rückhalt und Massenfluss durch die Membran ändern. Der MPI wird

dann auf Grundlage der veränderten Parameter neu errechnet. Liegt er nun über 0.35 ist eine vorteilhafte Abtrennung gefunden; liegt er weiter darunter, müssen die Betriebsparameter weiter geändert werden. Ist mit Parameteranpassung kein hinreichend hoher MPI erhältlich muss die Rezeptur für das trennaktive Material der Membran angepasst werden. Mit der angepassten Rezeptur kann dann ein Membranmuster hergestellt und im relevanten Stoffsystem vermessen werden um die grundlegenden Kenngrößen Rückhalt und Permeabilität unter Standard-Prozessbedingungen zu bestimmen. Es ist auch möglich, durch Iteration den MPI weiter zu optimieren auf Werte über 0.35, besser noch auf 0.5 oder sogar auf über 0.75. Denn die Wirtschaftlichkeit steigt mit wachsendem MPI. Die Auslegung zielt mithin darauf ab, einen möglichst hohen MPI zu erreichen.

Bei der Auslegung ist eine Anpassung des Permeatmassenstroms m' an die Produktionsbedürfnisse (Anlagenkapazität, Anpassung an Mehr- oder Mindermengen) von Bedeutung. Bei durch das Membranmaterial vorgegebener Permeabilität $P := m'/(A * \Delta p)$, kann dieser Massenstrom durch Anpassung der Membranfläche A oder des Transmembrandruckes $\Delta p$ angepasst werden. Ist eine Anpassung nicht möglich, so kann durch Wahl eines anderen Membranmaterials mit entsprechend höherer oder geringerer Permeabilität Abhilfe geschaffen werden. Hintergrund ist, dass die sich aus der Membranfläche unmittelbar die Kosten für das Membranmaterial ergeben. Sofern das Membrantrennverfahren zur Erzielung eines MPI über 0.35 unverhältnismäßig große (also teure) Membranflächen benötigt, ist ein Abbruchkriterium erfüllt oder ein anders Membranmaterial muss gewählt werden. Auch der maximale Transmembrandruck ist durch die Membran vorgegeben, da bestimmte Membranen ab einem gewissen Transmembrandruck durchbrechen. Entscheidet ist hier das Stützmaterial, auf welchem das trennaktive Material aufgebracht ist.

Die Dimensionierung der Nanofiltration und die Optimierung des MPI wird in der Regel computerimplementiert ausgeführt werden. Ein entsprechendes Computerprogramm, weist Instruktionen auf die zur Durchführung des Auslegungsverfahrens eingerichtet ist. Das Computerprogramm kann als eigenständige Software programmiert sein oder in eine bestehende Simulationssoftware integriert sein.

Das Computerprogramm kann verkörpert sein in einem Computerprogrammprodukt, welches ein Computerprogramm auf einem elektronischen Signal und/oder auf einem computerlesbaren Medium aufweist, bei dem nach dem Laden des Computerprogramms in einen Computer dieser zur Durchführung des Verfahrens Auslegungsverfahrens eingerichtet ist.

Das Ergebnis der erfolgreichen Planung der Nanofiltration unter Berücksichtigung des MPI ist ein Verfahren zur Abtrennung bzw. Rückhaltung eines Homogenkatalysators oder zumindest von Bestandteilen oder Abbauprodukten desselben aus einem Reaktionsgemisch mit Hilfe organophiler Nanofiltration, bei dem ein Reaktionsgemisch umfassend ein flüssiges organisches Lösemittel (in der Regel ist das eine Mischung aus Einsatzstoffen, sowie Ziel- und Nebenprodukten) und darin gelöst ein Homogenkatalysator und/oder Bestandteile und/oder Abbauprodukte des Homogenkatalysators auf eine Membran gegeben und daran getrennt wird in ein Retentat und in ein Permeat, wobei der Homogenkatalysator bzw. seine Bestandteile und/oder Abbauprodukte in dem Retentat dergestalt angereichert wird, dass die Konzentration des Homogenkatalysators bzw. seiner Bestandteile und/oder Abbauprodukte in dem Retentat größer ist als in dem Permeat und in dem Reaktionsgemisch, und wobei es sich bei dem Homogenkatalysator um eine metallorganische Komplexverbindung mit einem Metall als Zentralatom handelt, wobei die Abtrennung bei einem dimensionslosen Membran-Leistungsindikator MPI von größer als 0.35 erfolgt, und wobei der Membran-Leistungsindikator wie folgt definiert ist:

$$MPI := 0.5\ R^{S/7}\ (1+P)^{S/(100*R)} - 1/(10*P)$$

worin R für den dimensionslosen Rückhalt der Membran hinsichtlich des Metalls (bzw. des kostenrelevanten Bestandteils des Homogenkatalysators und/oder dessen Abbauprodukte) und P für die Permeabilität der Membran in $kg/(m^2 *bar*h)$ steht und worin S für einen angenommenen oder den gegenwärtigen Marktpreis des Metalls (bzw. des kostenrelevanten Bestandteils des Homogenkatalysators und/oder dessen Abbauprodukte) in Euro/g steht.

Dies betrifft den laufenden Betrieb einer Homogenkatalysatorabtrennung aus einem Reaktionsgemisch mit Hilfe einer Nanofiltration, welche unter Berücksichtigung des MPI ausgelegt wurde und dementsprechend unter Einhaltung eines MPI über 0.35 oder besser arbeitet. Bestenfalls wird dieser MPI bei dem gegenwärtigen, tatsächlichen Marktpreis eingehalten. Es kann aber auch sein, dass der MPI nur bei einem zuvor angenommenen Marktpreis des Katalysator-Metalls (bzw. des kostenrelevanten Bestandteils des Homogenkatalysators) eingehalten wird. Insoweit besteht das Risiko darin, dass der tatsächliche Marktpreis des Metalls (bzw. des kostenrelevanten Bestandteils des Homogenkatalysators) sich von dem in der Planung angenommen Wert zu stark fortentwickelt, wodurch der MPI ungünstig werden kann.

Da sich der Membran-Leistungsindikators MPI nicht nur bei der Planung, sondern auch bei Durchführung einer organophilen Nanofiltration zur Abtrennung von Homogenkatalysator aus Reaktionsgemischen gemäß Anspruch 1 nutzen lässt, ist die Verwendung des MPI bei der Ausübung eines solchen Verfahrens erfindungsgegenständlich.

Eine weitere Ausführungsform der Erfindung besteht in der Vorrichtung zur Abtrennung von Homogenkatalysator und/oder zumindest von Bestandteilen und/oder Abbauprodukte desselben aus einem Reaktionsgemisch im Wege der organophilen Nanofiltration, mit einer Membran, die einen Zulauf für einen Feed, einen Ablauf für ein Retentat und einen Ablauf für ein Permeat aufweist, dadurch gekennzeichnet, dass die Membran so bemessen ist, dass ein dimensionsloser Membran-Leistungsindikator MPI im bestimmungsgemäßen Betrieb der Vorrichtung mindestens 0.35 beträgt, wobei der Membran-Leistungsindikator wie folgt definiert ist:

$$MPI := 0.5\, R^{S/7}\, (1+P)^{S/(100*R)} - 1/(10*P)$$

worin R für den dimensionslosen Rückhalt der Membran hinsichtlich des Metalls (bzw. des kostenrelevanten Bestandteils des Homogenkatalysators und/oder dessen Abbauprodukte) und P für die Permeabilität der Membran in $kg/(m^{2*}bar*h)$ steht und worin S für einen angenommenen oder den gegenwärtigen Marktpreis des Metalls (bzw. des kostenrelevanten Bestandteils des Homogenkatalysators und/oder dessen Abbauprodukte) in Euro/g steht, und wobei es sich bei dem Homogenkatalysator um eine metallorganische Komplexverbindung mit dem besagten Metall als Zentralatom handelt. Die Liganden der metallorganischen Komplexverbindung bzw. deren Abbauprodukte kommen dabei ebenfalls als kostenrelevanter Bestandteils des Katalysators in Betracht.

Um eine solche Vorrichtung auch langfristig (d.h. bei möglicherweise schwankenden Rh-Preisen) in einem erfindungsgemäßen Zustand, also bei einem MPI von 0,35 oder höher betreiben zu können, sind verschiedene Weiterbildungen der Erfindung nötig.

Eine solche erweiterte Ausführungsform der Erfindung ist dergestalt beschaffen, dass jedesmal wenn aus Gründen der Materialalterung, bzw. -ermüdung (die Membranmaterialien unterliegen einem Alterungsverhalten, welches einen regelmäßigen Ersatz der Membranen nötig macht) ein Wechsel aller oder zumindest eines Teils der Membranmodule notwendig wird, eine aktuelle Betrachtung und Einschätzung des Rhodiumpreises (bzw. des Preise des Liganden) durchgeführt wird. Weichen die aktuellen und für den nächsten Betriebszeitraum erwarteten Rh-Preise dergestalt von den historischen Werten ab, dass die Anlage außerhalb des vorteilhaften MPI-Bereichs betriebe würde, so wird für die neu einzusetzen Membranmodule ein anderes Membranmaterial verwendet, das ein geeignetes Wertepaar für Rückhalt und Permeabilität aufweist, so dass der MPI im Betrieb wieder in den vorteilhaften Bereich gebracht werden kann.

Um auf Veränderungen von Betriebsparametern oder des Metallpreises reagieren zu können, sieht die erfindungsgemäße Vorrichtung es vor, diese mit einer Regelung zu versehen, die dafür eingerichtet ist, zumindest einen Betriebsparameter der Vorrichtung so zu steuern, dass der Ist-Wert des Membran-Leistungsindikators der Vorrichtung auf einen größer oder gleich 0.35 eingestellten Soll-Wert des Membran-Leistungsindikators geregelt wird. Durch die Regelung wird sichergestellt, dass die Vorrichtung auch bei sich ändernden Betriebsbedingungen bei vorteilhaften MPI arbeitet.

Grundsätzlich ist die Erfindung nicht auf bestimmte Membranmaterialien beschränkt. Es zeigt sich allerdings, dass gerade solche Membranen, die eine trennaktive Schicht aus Siliconacrylat aufweisen, durch besonders hohe Membran-Leistungsindikatoren auffallen. Mithin werden besonders bevorzugt Siliconacrylat-Membranen zur Abtrennung von Homogenkatalysatoren aus Reaktionsgemischen eingesetzt. Unter einem Siliconacrylat ist in diesem Zusammenhang eine Verbindung der folgenden Struktur zu verstehen:

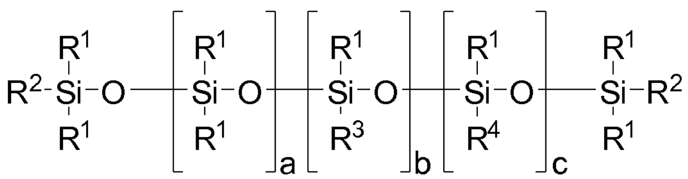

wobei gilt:

a = 25 - 500,

b = 1 - 25,

c = 0 - 20,

R1 = unabhängig voneinander gleiche oder verschiedene Alkyl- oder Arylreste mit 1 bis 30 C-Atomen die gegebenenfalls Ether- und/oder Ester- und/oder Epoxy- und/oder Alkohol-Funktionen tragen,

R2 = unabhängig voneinander gleiche oder verschiedene Reste aus der Gruppe: R1, R3 und R4,

R3 = unabhängig voneinander gleiche oder verschiedene organische Reste die ein oder mehrere Acrylatgruppen tragen,

R4 = gleiche oder verschiedene Polyether-Reste.

Ein derartiges trennaktive Membranmaterial wird auf eine Trägerschicht aufgebracht um eine entsprechende Membran zur erhalten (sog. Kompositmembran). Dies geschieht durch Dip-Coating. Eine derartige Siliconacrylat-Membran ist in WO2011/067054A1 näher beschrieben. Die Besonderheit dieser Membranen besteht darin, dass die verwendeten Siliconacrylate die nicht an den Kettenenden, sondern seitenständig modifiziert wurden. Bei dem vorzugsweise eingesetzten Membranmaterial handelt es sich also genauer gesagt, um seitenständig modifiziertes Siliconacrylat. Dabei kann es vorteilhaft sein, mehrere trennaktive Schichten verschiedener Silikonacrylate aufzubringen. Außerdem kann durch das Aufbringen zweier oder mehrerer verschiedener Silikonacrylate in unterschiedlichen Mischungsverhältnissen die Eigenschaften der Membran an den Bedarf des Prozesses angepasst werden.

Die Verwendung der oben beschriebenen Siliconacrylate als Reinstoffe oder Mischungen zur Ausbildung der trennaktiven Schicht hat einen besonderen Vorteil: das für die Berechnung des dimensionslosen Membranleistungs-Indikators MPI benötigte Wertepaar aus Rückhalt und Permeabilität kann über einen weiten Bereich eingestellt werden. Figur 1 zeigt dieses Wertepaar für verschiedene Membrantypen: eine kommerzielle Vergleichsmembran mit einer Trennschicht aus Polydimethylsiloxan (PDMS), dargestellt mit dem Symbol Raute ◊, eine Reihe von drei verschiedenen Siliconacrylat-Membranen deren Trennschicht aus jeweils einem Siliconacrylat besteht, dargestellt mit dem Symbol Dreieck ▲, sowie eine Reihe von vier Siliconacrylat-Membranen deren Trennschicht jeweils auf einer Mischung von drei Siliconacrylate besteht, dargestellt mit dem Symbol Quadrat ■.

Auf der horizontalen Achse des in Figur 1 dargestellten Graphen ist die Permeabilität in [kg / h $m^2$ h] aufgetragen. Auf der vertikalen Achse des in Figur 1 dargestellten Graphen ist der Rhodium - Rückhalt in [%] aufgetragen. Dem Diagramm der Figur 1 sind auch die jeweils berechneten Membranleistungsindikatoren (MPI) zu entnehmen. Diese wurden gemäß der angegebenen Formel allesamt bei einem Rhodiumpreis von 75 Euro/g berechnet. Die MPI-Werte sind in dem Diagramm an den Messpunkten eingetragen.

Es wird deutlich, dass die Siliconacrylat-Membranen (Symbole Dreieck ▲ und Quadrat ■), und insbesondere solche, die Mischungen von Siliconacrylaten als trennaktive Schicht aufweisen Symbol Quadrat ■), in dem erfindungsgemäßen Verfahren besonders vorteilhaft sind, da sie hohe MPI-Werte erzielen. Zu der Charakterisierung und Herstellung der vorzugweise eingesetzten, seitenständig modifizierten Siliconacrylate wird auf die WO2011/067054A1 verwiesen.

Zu dieser Schrift ist allerdings anzumerken, dass dort die Bewertung der Membranen auf Basis von Modellsystemen getroffen worden ist, wie zum Beispiel Polystyrole verschiedener Molekulargewichte in n-Heptanlösung. Da diese einfachen Modellsysteme in der Regel vollkommen andere Komponenten enthalten als die realen Anwendungssysteme, kann es im Verhalten der Membran zu Abweichungen kommen. Die Ursache liegt oft darin, dass die realen Systeme Veränderungen (zum Beispiel der Zersetzung von Katalysatorkomponenten) unterliegen und die Flüssigkeitsgemische sich chemisch und physikalisch ganz anders verhalten können als die in den Modellsystemen verwendeten Lösungsmittel.

Aus diesem Grund erweist es sich als zweckmäßig, für die Bewertung einer Membran nicht wie sonst üblich nur den erzielten Rückhalt einer Modellkomponente repräsentativer Molmasse zu berücksichtigen sondern vielmehr die Trennleistung der Membran in dem realen Anwendungssystem. Darüber hinaus sollte man bei der Bewertung berücksichtigen, dass die wirtschaftliche Trennleistung nicht nur durch den Rückhalt der Membran für die rückzuhaltende Komponente bestimmt wird, sondern auch durch den durchschnittlichen Fluss des zu permeierenden Mediums durch die Membran. Daher wird erfindungsgemäß für die Katalysatorrückhaltung in homogen katalysierten chemischen Prozessen eine Bewertung auf Basis des Membran-Leistungsindikators (MPI) vorgenommen.

Das Reaktionsgemisch, aus dem unter erfindungsgemäßer Berücksichtigung des Membran-Leistungsparameters der Homogenkatalysator abgetrennt wird, kann aus unterschiedlichen homogen katalysierten chemischen Reaktionen stammen. Neben der bereits ausführlich beschriebenen Hydroformylierung kann das Reaktionsgemisch auch aus einer Hydrocarboxylierung, Alkoxycarbonylierung, Methoxycarbonylierung, Hydrierung, oder Hydrocyanierung stammen. All diese Reaktionen werden nämlich üblicherweise mit einer metallorganischer Komplexverbindung homogen katalysiert, wobei als Zentralatome die folgenden Metalle in Betracht kommen: Cobalt, Rhodium, Iridium, Ruthenium, Palladium. Sofern das jeweilige Katalysatorsystem die Reaktion katalysiert, wird gemäß der Erfindung ein solches homogenes Katalysatorsystem aus dem aus der Reaktion stammenden Reaktionsgemisch abgetrennt.

**Beispiel 1 - Herstellung erfindungsgemäßer Siliconacrylatmembranen**

Siliconacrylat-Membranen, die den erfindungsgemäßen Anforderungen genügen können zum Beispiel besonders vorteilhaft mit Hilfe eines sogenannten Dip-Coating-Verfahrens hergestellt werden. Derartige Verfahren sind zum Beispiel in EP0330076A2 beschrieben. Bei derartigen Verfahren wird eine geeignete Unterstruktur durch Eintauchen einseitig mit einer Lösung von einem oder mehreren Siliconacrylaten, sowie ggf. Hilfsstoffen wie Vernetzern oder Fotoinitiatoren beschichtet. Aus dem anhaftenden Flüssigkeitsfilm wird das Lösungsmittel in einem Trockner verdampft. Der verbliebende Film aus Siliconacrylaten und Hilfsstoffen kann dann durch geeignete Methoden vernetzt werden, z.B. durch die Bestrahlung mit kurzwelliger elektromagnetischer Strahlung (z.B. Ultraviolettes Licht).

Figur 2 zeigt den schematischen Versuchsaufbau einer geeigneten Apparatur zur Herstellung erfindungsgemäßer Siliconacrylat-Membranen.

Im vorliegenden Fall wurde eine Ultrafiltrationsmembran vom Typ L6 der Firma GMT Membrantechnik GmbH als Unterstruktur 1000 verwendet, grundsätzlich eignen sich aber auch andere Ultrafiltrationsmembranen (z.B. auf Basis von Polyimid) ebenfalls für die Herstellung von Siliconacrylat-Membranen.

Die Unterstruktur wurde in eine Beschichtungsmaschine (wie sie in Figur 2 schematisch dargestellt ist) eingespannt und konnte mit Geschwindigkeiten zwischen 0,5 m/s und 5 m/s durch Eintauchen in das Polymerbad beschichtet werden. Das Tauchbad 2000 wurde mit einer Lösung der entsprechenden Siliconacrylate und ggf. Hilfsstoffe in Isopropanol befüllt. Die beschichtete Unterstruktur wurde in einem Heißlufttrockner 3000 getrocknet. Dieser muss wegen des abdampfenden entzündlichen Lösungsmittels explosionsgeschützt sein (Zulassung gemäß ATEX). Die angetrocknete Siliconacrylatschicht der Rohmembran wurde dann unter weitgehend sauerstofffreien Bedingungen (Überlagerung mit Stickstoff 5.0) durch Bestrahlung mit UV-Licht 4000 vernetzt und die fertige Membran zu einer Rolle 5000 gewickelt.

Für die weiteren Untersuchungen wurde eine Reihe von Siliconacrylat-Membranen wie oben beschrieben hergestellt. Für den Vergleich wurden außerdem einige Membranen mit anderen Trennaktiven Schichten herangezogen. Alle betrachteten Membranen sind in Tabelle 1 zusammengestellt.

Tabelle 1 Beispielmembranen

| Bez. | Trennaktives Polymer | Handelsname | Komponenten/ Zusammensetzung | | | Konz. | Geschwindigkeit |
|---|---|---|---|---|---|---|---|
| | | | (%) | (%) | (%) | % | m/min |
| A | Polydimethylsiloxan | ONF-2 (GMT Membrantechnik) | --- | --- | --- | --- | --- |
| B | Polyimid | Starmem 122 (Grace/UOP) | --- | --- | --- | --- | --- |
| C | Polyimid | Starmem 240 (Grace/UOP) | --- | --- | --- | --- | --- |
| D | Polyimid | Puramem 280 (Evonik Industries AG) | --- | --- | --- | --- | --- |
| E | Siliconacrylat | --- | XP8032 (100) | --- | --- | 2,5 | 2,8 |
| F | Siliconacrylat | --- | XP8032 (100) | --- | --- | 3,0 | 3,5 |
| G | Siliconacrylat | --- | XP8032 (100) | --- | --- | 1,25 | 4,2 |
| H | Siliconacrylat | --- | XP8032 (100) | --- | --- | 1,75 | 3,5 |
| I | Siliconacrylat | --- | RC702 (100) | --- | --- | 1,75 | 2,8 |
| J | Siliconacrylat | --- | RC702 (100) | --- | --- | 1,75 | 3,5 |
| K | Siliconacrylat | --- | XP8032 (70) | RC902 (30) | --- | 2,5 | 2,2 |

(fortgesetzt)

| Bez. | Trennaktives Polymer | Handelsname | Komponenten/ Zusammensetzung | | | Konz. | Geschwindigkeit |
|---|---|---|---|---|---|---|---|
| | | | (%) | (%) | (%) | % | m/min |
| L | Siliconacrylat | --- | XP8026 (60) | RC902 (30) | XP8028 (10) | 2,0 | 2,8 |
| M | Siliconacrylat | --- | XP8026 (50) | RC902 (40) | XP8028 (10) | 2,0 | 4,0 |
| N | Siliconacrylat | --- | XP8026 (50) | RC902 (40) | XP8028 (10) | 2,0 | 4,3 |
| O | Siliconacrylat | --- | XP8026 (50) | RC902 (40) | XP8028 (10) | 2,0 | 2,8 |
| P | Siliconacrylat | --- | XP8026 (50) | RC902 (40) | XP8028 (10) | 2,0 | 2,2 |

**Beispiel 2 - Bestimmung des MPI**

Dieses Beispiel beschreibt die Vorgehensweise zur Bestimmung des Membran-Leistungsindikators MPI. Die zu testende Membran wird in einem Querstromfiltrationsexperiment untersucht. Der prinzipielle apparative Aufbau für diese Versuche ist in Figur 3 dargestellt.

Die Versuche werden in der dargestellten Kreislaufapparatur durchgeführt. Ein zu untersuchendes Substanzgemisch, oder dessen Einzelkomponenten können über einen Zugang 100 in einen temperierbaren Rührkesselbehälter 200 gefüllt werden. Die Mischung wird hier während der gesamten Versuchsdauer durch Rühren homogenisiert. Bei Bedarf kann an dem Behälter ein Hilfsgas angeschlossen werden, welches zur Stabilisierung der Katalysatorkomplexe verwendet wird (in Figur 3 nicht dargestellt) Die in dem Behälter 100 vorliegende Mischung A kann nun über eine Druckerhöhungspumpe 300 auf den für die Trennung erforderlichen Vordruck auf der Retentatseite einer Membran 600 gebracht werden. Der Druck wird über ein Druckhalteventil 8 eingestellt. Die Mischung A wird nach der Pumpe mit einem Rückführstrom D der Membran 600 vereinigt und von einer Kreislaufpumpe 400 in eine Membranzelle 500 befördert. In dieser Zelle strömt die Mischung quer an der zu testenden Membran 600 vorbei. Die Geometrie der Membranzelle gibt dabei die verfügbare Membranfläche vor. Ein Teil der Mischung permeiert dabei entlang eines Druckgefälles durch die Membran 600 und verlässt die Membranzelle als sogenanntes Permeat F. Der Druck auf der Permeatseite wird durch ein Druckhalteventil 900 eingestellt und liegt immer unterhalb des Druckes auf der Retentatseite der Membran. Der Druckunterschied zwischen den beiden Drücken wird als Transmembrandruck $\Delta p$ bezeichnet. Das Permeat F wurde bei Durchtritt durch die Membran von der zurückzuhaltenden Katalysatorkomponente abgereichert und wird in den Rührbehälter 200 zurückgeführt. Der Teil der Mischung B, der nicht durch die Membran strömt, verlässt die Membranzelle als katalysatorreiches Retentat C. Ein Teil des Retentats wird mit Hilfe eines Überströmventils 700 als Rückführstrom D vor die Kreislaufpumpe 400 zurückgeführt. So kann eine konstante und hohe Durchströmung der Membranzelle 500 bewirkt werden, was einer Konzentrationspolarisation an der Membran entgegenwirkt. Der übrige Teil des Retentats E wird in den Behälter 200 zurückgeführt.

Zur Bestimmung der Permeabilität P muss der Massenstrom m' des Permeatstromes F bestimmt werden, dies kann durch eine handelsübliche Massenflussmessung erfolgen. Des Weiteren sind die Drucke auf der Retentat- und auf der Permeatseite der Membran durch handelsübliche Druckmessgeräte zu messen. Aus den Werten ergibt sich der Transmembrandruck $\Delta p$ gemäß

$$\Delta p = P_{\text{Rentatseite}} - P_{\text{Permeatseite}}$$

Aus diesen Werten und der bekannten Membranfläche kann die Permeabilität P bestimmt werden:

$$P = m' / (A * \Delta p)$$

Zur Bestimmung des Rückhalts R muss die Massenkonzentration (bzw. der Massenanteil) der zurückzuhaltenden Komponente (repräsentiert durch das Zentralatom im Katalysatorkomplex) im Retentatstrom $w_R$ und im Permeatstrom $w_P$ analysiert werden. Hierzu können durch dem Fachmann bekannte Techniken Proben aus der Anlage entnommen werden und eine geeigneten Analytik zugeführt werden. Für die Bestimmung von Metallgehalten in organischen Medien eignet sich zum Beispiel eine Analyse mittels Massenspektrometrie mit induktiv gekoppeltem Plasma (englisch: inductively coupled plasma mass spectrometry, ICP-MS). Aus den so bestimmten Metallgehalten der beiden Ströme ergibt sich der Rückhalt gemäß

$$R := 1 - w_P/w_R$$

Dabei ist zu beachten, dass Rückhalt und Permeabilität systemspezifisch sind, also sowohl vom Material und der Beschaffenheit der Membran, als auch von dem zu trennenden Stoffsystem abhängen. Da die spezifische Permeabilität außerdem eine gewisse Druckabhängigkeit und eine mitunter ausgeprägte Temperaturabhängigkeit aufweist, ist sicherzustellen, dass die Permeabilität einer Membran nur unter für den späteren Trennprozess relevanten Bedingungen zu bestimmen ist. Zu diesem Zweck erweist es sich als günstig, die Membran bei verschiedenen Transmembrandrücken und Temperaturen zu vermessen. Sodann kann aus den erhaltenen Wertepaaren, wie im Folgenden angegeben der MPI bestimmt werden und so die optimalen Bedingungen für jede Membran ausgewählt werden (maximaler MPI).

Für die Bestimmung des MPI wird außer dem Rückhalt und der Permeabilität noch der Marktpreis S des jeweiligen Metalls nötig. Dieser kann geeigneten Quellen entnommen werden, üblicherweise werden die aktuellen und historischen Edelmetallpreise von diversen Edelmetallhändlern zur Verfügung gestellt und können über deren Internetseiten abgefragt werden, beispielsweise von der Firma Heraeus unter der URL http://heraeus-edelmetallhandel.de/de/marktinformationen/edelmetallpreise/edelmetallpreise.aspx Der Membran-Leistungsindikator MPI kann nun aus der Permeabilität in kg/m$^2$/bar/h, dem dimensionslosen Rückhalt und dem Marktpreis des Metalls in Euro/g wie folgt berechnet werden:

$$MPI := 0.5\ R^{S/7}\ (1+P)^{S/(100*R)} - 1/(10*P)$$

Verschiedene Membranen sollten dabei immer mit ihrem optimalen MPI verglichen werden (abhängig von Temperatur und Transmembrandruck, s.o.). Außerdem sollten nur MPI-Werte miteinander verglichen werden, die bei gleichem Metallpreis S berechnet wurden.

Exemplarisch wird im Folgenden die Bestimmung des MPI für beispielhafte Membranen A bis P bestimmt, die für die Rückhaltung eines Homogenkatalysators in einem

Hydroformylierungsprozess eingesetzt werden. Als Homogenkatalysator soll ein für diesen Zweck üblicher metallorganischer Komplex umfassend zumindest ein Rh-Zentralatom verwendet werden. Eine Komplexspezies, die in dem System zum Beispiel mittels Operando-IR-Spektroskopie nachgewiesen werden kann ist [HRh(CO)3L], wobei L für einen Organophosphitliganden steht wie zum Beispiel das handelsübliche Triarylphosphit Alkanox 240. Hydroformyliert werden C8-Olefine zu C9-Aldehyden.

Im Rahmen des Versuchs wurde der Rührbehälter 2 der in Figur 3 gezeigten Anlage mit einem Gemisch gefüllt, welches einem aus einer Hydroformylierung stammenden Reaktionsgemisch entspricht. Hierzu wurde zu einer Mischung, die im folgenden INAL genannt wird, mit der Zusammensetzung:

| | |
|---|---|
| Isononanal | > 85 Gew.-% |
| Isononanol | < 3 Gew.-% |
| Di-n-buten | < 5 Gew.-% |
| C8-Alkane | < 1 Gew.-% |
| Hochsiedender | < 10 Gew.-% |

so viel (Acetylacetonato)dicarbonylrhodium(I) (z.B. CARAC, der Firmen Umicore oder Heraeus) und Tris(2,4-di-tert-butylphenyl)phosphit (z.B. Alkanox 240 der Firma Addivant) gegeben, dass sich ein Rhodiumgehalt von 75 bis 90 mg/kg Gemisch einstellte. Der Phosphitligand Alkanox 240 wurde in einer Menge zugefügt, dass ein molares Phosphor-Rhodium-Verhältnis von 5:1 eingestellt wurde. Um eine dem technisch anfallenden Katalysator entsprechende Komponente zu erzeugen, wurde diese Mischung für einige Stunden unter einem Synthesegasüberdruck und erhöhter Temperatur gerührt, bevor mit der Durchströmung der Membrantestzelle begonnen wurde. Für den Membrantest wurden die Temperatur auf 40 °C und der Transmembrandruck auf 30 bar eingestellt. Mit Hilfe der Kreislaufpumpe 4 und dem Überströmer

7 wurde ein Kreislaufstrom von ca. 200 L/h auf der Retentatseite eingestellt. Die Menge des durch die Membran strömenden Permeats wurde kontinuierlich gemessen. Nach Einstellung eines stabilen Betriebszustandes wurden wiederholt Proben aus dem Retentat C und dem Permeat F gezogen und mittels ICP-MS auf den Rhodiumgehalt hin analysiert. Die für die verschiedenen Membranen bestimmten optimalen Werte für Permeabilität und Rhodiumrückhalt sind der folgenden Tabelle zu entnehmen. Aus diesen Werten wurde der MPI für vier verschiedene (willkürliche, aber historisch vorgekommene) Rhodiumpreise berechnet: 25, 50, 75 und 100 Euro pro Gramm Rhodium. Die Ergebnisse sind in Tabelle 2 wiedergegeben.

Tabelle 2: Bestimmte Membran-Leistungsindikatoren für verschiedene Membranen

| Bez. | Rh-Rückhalt | Permeabilität | MPI | | | |
|---|---|---|---|---|---|---|
| | [%] | [kg/(m2 h bar)] | Rh @ 25 EUR/g | Rh @ 50 EUR/g | Rh @ 75 EUR/g | Rh @ 100 EUR/g |
| A | 93,8 | 0,86 | 0.35 | 0.30 | 0.30 | 0.25 |
| B | 98,5 | 0,30 | 0.15 | 0.20 | 0.20 | 0.20 |
| C | 97,4 | 0,40 | 0.25 | 0.25 | 0.25 | 0.25 |
| D | 90,0 | 0,30 | 0.05 | 0.00 | 0.00 | 0.00 |
| E | 74,7 | 2,66 | 0.25 | 0.10 | 0.05 | 0.00 |
| F | 97,3 | 0,50 | 0.30 | 0.30 | 0.30 | 0.30 |
| G | 93,9 | 1,13 | 0.40 | 0.40 | 0.40 | 0.35 |
| H | 94,4 | 0,95 | 0.40 | 0.35 | 0.35 | 0.35 |
| I | 89,6 | 1,49 | 0.35 | 0.30 | 0.25 | 0.20 |
| J | 95,1 | 0,83 | 0.35 | 0.35 | 0.35 | 0.35 |
| K | 91,1 | 1,55 | 0.40 | 0.35 | 0.35 | 0.30 |
| L | 94,0 | 1,32 | 0.45 | 0.45 | 0.45 | 0.45 |
| M | 85,0 | 2,83 | 0.40 | 0.30 | 0.25 | 0.20 |
| N | 89,5 | 1,96 | 0.40 | 0.35 | 0.35 | 0.30 |
| O | 95,2 | 1,08 | 0.40 | 0.45 | 0.45 | 0.45 |
| P | 93,7 | 1,72 | 0.45 | 0.50 | 0.50 | 0.50 |

**Beispiel 3**

Dieses Beispiel beschreibt die Simulation eines Hydroformylierungsprozesses gemäß Figur 4. Figur 4 zeigt das Schaltbild einer erfindungsgemäßen Anlage mit welcher das erfindungsgemäße Verfahren durchgeführt werden kann. Wie üblich ist das Schaltbild vereinfacht, um die Lesbarkeit zu verbessern. Selbstverständliche Anlagenkomponenten wie Ventile, Pumpen und dergleichen sind nicht dargestellt.

Kernstück der Anlage bildet ein Hydroformylierungsreaktor 1. Hierin findet die Hydroformylierungsreaktion statt. Dabei wird ein Olefin 2 mit Synthesegas 3 - eine Mischung aus Kohlenmonoxid und Wasserstoff - in Gegenwart eines homogen gelösten Katalysators zu entsprechenden Aldehyden mit einem Kohlenstoffatom mehr umgesetzt. Bei dieser Reaktion handelt es sich um eine Gas/Flüssigphasen-Reaktion, bei welcher das Olefin und die Reaktionsprodukte in der flüssigen Phase vorliegen, währenddessen ein Teil des Synthesegases 3 die Gasphase bildet und ein anderer Teil des Synthesegases in der flüssigen Phase gelöst ist. Ebenfalls in der flüssigen Phase gelöst ist ein homogener Katalysatorkomplex.

Als Reaktorbauart kommen grundsätzlich solche Apparate in Betracht, die eine Gas-Flüssigphasenreaktion erlauben. Bevorzugt wird ein Blasensäulen-Reaktor eingesetzt. Blasensäulen-Reaktoren sind im Stand der Technik allgemein bekannt und werden ausführlich im ULLMANN beschrieben: Deen, N.G., Mudde, R.F., Kuipers, J.A.M., Zehner, P. and Kraume, M.: Bubble Columns. Ullmann's Encyclopedia of Industrial Chemistry. Published Online: 15 January 2010. DOI: 10.1002/14356007.b04_275.pub2

Für die Simulation kann aber aus Gründen der Einfachheit ein ideal durchmischter Rührkesselreaktor angenommen werden, da für die hier zu betrachtende Fragestellung die Hydroformylierungsreaktion von nachrangiger Bedeutung ist.

In der Hydroformylierung bilden sich neben den gewünschten Aldehyden in anschließender Folgereaktion auch die entsprechenden Alkohole sowie Hochsieder. Zu den Hochsiedern gehören unter anderem Dimere, Trimere, Aldolprodukte, Tishchenko-Produkte, Ester und Ether. Die Hochsiederbildung in der Reaktion ist unerwünscht, da sie zu Ausbeuteverlusten führt, ist aber reaktionstechnisch nicht völlig zu vermeiden. Die Hochsieder müssen deswegen entsprechend ihrer Bildungsrate aus dem Prozess ausgeschleust werden. Die Bezeichnung Hochsieder rührt daher, dass diese Substanzen einen höheren Siedepunkt aufweisen als der Aldehyd, weswegen sich die Hochsieder am Sumpf der thermischen Trenneinheit bzw. der Destillation hinter der Hydrierung ansammeln. Demgegenüber gehören zu den Leichsiedern Olefine, Alkane und Aldehyde, die in der Hydroformylierung oder der Hydrierung gebildet werden bzw. bereits in dem Olefingemisch vorhanden sind.

Aus dem Reaktor 1 wird ein flüssiger Hydroformylierungsaustrag 4 abgezogen, welcher neben dem gewünschten Aldehyd auch unumgesetztes Olefin, in der Flüssigkeit gelöstes Synthesegas, das homogen gelöste Katalysatorsystem, weitere Leichtsieder und die Hochsieder enthält. Sofern ein optionales Lösungsmittel eingesetzt wird, gehört dieses zu den Leichtsiedern.

In einem ersten Wärmetauscher 5 wird der Hydroformylierungsaustrag 4 auf eine Temperatur von etwa 40 bis 50 °C abgekühlt. In einem ersten Entgaser 9a wird der Hydroformylierungsaustrag 4 auf etwa 0.5 MPa entspannt und das dabei ausperlende Synthesegas 3 in den Reaktor 1 zurückgeführt. Sodann wird der Hydroformylierungsaustrag 4 einer ersten Membrantrenneinheit 6 aufgegeben. Bei der Membrantrenneinheit 6 handelt es sich um eine mehrstufige Abtriebskaskade, wie sie aus dem Stand der Technik (z.B. DE102013203117 A1) bekannt ist Für den funktionalen Zusammenhang genügt es aber, die erste Membrantrenneinheit 6 wie eine Einzelmembran aufzufassen.

Der anströmende Hydroformylierungsaustrag 4 wird in der ersten Membrantrenneinheit 6 in einem Produktstrom 7 und in einen Reaktorrücklauf 8 aufgetrennt, wobei das im Hydroformylierungsaustrag 4 enthaltene Katalysatorsystem im Reaktorrücklauf 8 angereichert wird. Der Produktstrom 7 stellt dabei das Permeat der ersten Membrantrenneinheit 6 dar, währenddessen der Reaktorrücklauf 8 ihr Retentat bildet. Da die erste Membrantrenneinheit 6 das Katalysatorsystem deutlich langsamer passieren lässt als die übrigen Bestandteile des Hydroformylierungsaustrags 4, reichert sich im Reaktorrücklauf 8 das Katalysatorsystem an. Die Membrantrenneinheit 6 wird vorzugsweise so gefahren, dass sich etwa drei Viertel des aus dem Hydroformylierungsreaktors 1 ausgetragenen Katalysatorsystems im Reaktorrücklauf 8 findet. Der Rückhalt der ersten Membrantrenneinheit beträgt hinsichtlich des Katalysatorsystems somit 75 %. Dafür sind folgende Betriebsparameter einzuhalten:

Die Membrantemperatur liegt zwischen 20 und 160°C, bevorzugt zwischen 25 und 90°C und besonders bevorzugt zwischen 30 und 60°C. In den Konzentratloops kann eine um 10 bis 30 K höhere Temperatur vorteilhaft sein. Die transmembrane Druckdifferenz liegt zwischen 1 und 10 MPa, bevorzugt zwischen 1.5 und 5 MPa. Besonders bevorzugt wird die Membran bei etwa 2.5 bis 3.5 MPa Transmembrandruck betrieben. Die bevorzugt eingesetzte Membranmodulbauweise ist das Spiralwickelelement.

Vorzugsweise werden Membranen eingesetzt, die eine trennaktive Schicht aus einem Material, ausgewählt aus Celluloseacetat, Cellulosetriacetat, Cellulosenitrat, regenerierter Cellulose, Polyimiden, Polyamiden, Polyetheretherketonen, sulfonierten Polyetheretherketonen, aromatischen Polyamiden, Polyamidimiden, Polybenzimidazolen, Polybenzimidazolone, Polyacrylnitril, Polyarylethersulfone, Polyester, Polycarbonaten, Polytetrafluorethylen, Polyvinylidenfluorid, Polypropylen, endständig oder seitenständig organomodifiziertem Siloxan , Polydimethylsiloxan, Silicone, ,Polyphosphazene, Polyphenylsulfide, Polybenzimidazole, 6.6 Nylon®, Polysulfone, Polyaniline, Polypropylene, Polyurethane, Acrylonitril/Glycidylmethacrylat (PANGMA), Polytrimethylsilylpropyne, Polymethylpentyne, Polyvinyltrimethylsilan, Polyphenylenoxid, alpha-Aluminiumoxide, gamma-Aluminiumoxide, Titaniumoxide, Siliciumoxide, Zirconiumoxide, mit Silanen hydrophobisierte keramische Membranen, wie sie in EP 1 603 663 B1 beschrieben sind, Polymere mit intrinsischer Mikroporösität (PIM) wie PIM-1 und anderen, wie sie z. B. in EP 0 781 166 und in "Membranes" von I. Cabasso, Encyclopedia of Polymer Sience and Technlogy, John Wiley and Sons, New York, 1987, beschrieben werden, aufweisen. Die oben genannten Stoffe können insbesondere in der trennaktiven Schicht ggf. durch Zugabe von Hilfsstoffen vernetzt vorliegen oder als so genannte Mixed Matrix Membranen mit Füllstoffen wie z.B. Carbon Nano Tubes, Metal Organic Frameworks oder Hollow Spheres sowie Partikel von anorganischen Oxiden oder anorganische Fasern, wie z. B. Keramik- oder Glasfasern versehen sein.

Bevorzugt werden Membranen eingesetzt, die als trennaktive Schicht eine Polymerschicht aus Polydimethylsiloxan oder Polyimid aufweisen, oder es werden Membranen eingesetzt, deren trennaktive Schicht aus endständig oder seitenständig organomodifiziertem Siloxan (z.B. Siliconacrylte) besteht.

Neben den oben genannten Materialien können die Membranen weitere Materialien aufweisen. Insbesondere können die Membranen Stütz- oder Trägermaterialien aufweisen, auf die die trennaktive Schicht aufgebracht ist. Bei solchen Verbundmembranen liegt neben der eigentlichen Membrane noch ein Stützmaterial vor. Eine Auswahl von Stützmaterialien beschreibt

EP 0 781 166, auf welches explizit verwiesen wird.

Eine Auswahl von kommerziell erhältlicher Lösungsmittel stabiler Membranen sind die MPF und Selro Serien von Koch Membrane Systems, Inc., unterschiedliche Typen von Solsep BV, die Starmem™ Serie von Grace/UOP, die

DuraMem™ und PuraMem™ Serie von Evonik Industries AG, die Nano-Pro Serie von AMS Technologies, die HITK-T1 von IKTS, sowie oNF-1, oNF-2 und NC-1 von der GMT Membrantechnik GmbH und die inopor®nano Typen der Inopor GmbH.

Das Retentat der ersten Membrantrenneinheit 6 - hier als Reaktorrücklauf 8 oder auch als primäres Rezyklat bezeichnet - enthält neben der hohen Rhodiumkonzentration die übrigen Stoffe des Hydroformylierungsaustrags, nämlich Aldehyd, Olefin, gelöstes Synthesegas, weitere Leichtsieder und Hochsieder. Der Reaktorrücklauf 8 wird in den Hydroformylierungsreaktor 1 zurückgeführt. Der Reaktorrücklauf braucht dabei nicht - wie in der Zeichnung vereinfacht dargestellt - zusammen mit dem Frisch-Olefin 2 und dem frischen Synthesegas 3 in den Reaktor 1 eingespeist werden. Es ist durchaus denkbar, diese 3 Ströme an unterschiedlichen Stellen getrennt in den Hydroformylierungsreaktor 1 einzuspeisen.

Um zu verhindern, dass das Katalysatorsystem in der ersten Membrantrenneinheit 6 seine Aktivität verliert, wird dieser Membrantrennschritt unter Einhaltung eines Mindest-CO-Partialdampfdruckes durchgeführt. Dieser sollte, wie in EP1931472B1 geschildert, mindestens 100 kPa betragen. Aus diesem Grund erfolgt die Entspannung in Entgaser 9a nicht vollständig sondern nur bis auf 0.5 MPa. Das gelöste Synthesegas soll nämlich erst hinter der Membran entfernt werden. Hierfür wird der Produktstrom 7 in einem zweiten Entgaser 9b auf Atmosphärendruck entspannt. Das im Permeat der ersten Membrantrenneinheit verbliebene Synthesegas entweicht dabei vollständig und wird aus der Anlage abgeführt.

Sodann wird der entgaste Produktstrom 7 in eine thermische Trenneinheit 10 überführt. Dabei handelt es sich einfachstenfalls um eine Destillationskolonne, bevorzugt jedoch um eine Kombination aus einem Dünnschichtverdampfer und einem Fallfilmverdampfer oder einer Kombination von zwei oder drei Fallfilmverdampfern.

In der thermischen Trenneinheit 10 wird der Produktstrom 7 unter Wärmeeinwirkung verdampft. Hierfür wird am Fallfilmverdampfer eine Sumpftemperatur von 90°C eingestellt; die Sumpftemperatur am Dünnschichtverdampfer beträgt 100°C. Unterstützt wird die Verdampfung durch ein angelegtes Vakuum von jeweils etwa 30 hPa. Auf diese Weise verdampft mehr als 90 % der mit dem Produktstrom 7 in die thermische Trenneinheit 10 eingebrachten Masse. Diese dampfförmige Masse bildet das Kopfprodukt 11 der thermischen Trenneinheit. Da die eingebrachten Komponenten unterschiedliche Siedepunkte aufweisen, findet durch die Verdampfung nicht nur eine rein quantitative Trennung des Produktstroms 7 statt sondern auch eine qualitative: Aufgrund des niedrigeren Siedepunkts werden Aldehyd, Alkohol und die übrigen Leichtsieder bevorzugt im Kopfprodukt 11 angereichert. Die nicht verdampften Komponenten bilden ein flüssiges Sumpfprodukt 12, welches im Wesentlichen aus Hochsiedern, Aldehyd und Katalysatorsystem besteht, wobei Aldehyd und Hochsieder etwa denselben Gewichtsanteil ausmachen. Die Betriebsbedingungen der thermischen Trenneinheit sind so gewählt, dass sich vorzugsweise 95 % der mit dem Produktstrom 7 eingebrachten Aldehyde im Kopfprodukt 11 finden. Maximal 5 % der mit dem Produktstrom 7 eingebrachten Aldehyde verbleiben im Sumpf 12. Der als Kopfprodukt 11 abgezogene, die Wertprodukte enthaltende, große Massenstrom wird sodann durch einen ersten Adsorber 13 gefahren. Die Funktion des Adsorbers 13 besteht darin, Restmengen von Katalysator, insbesondere Edelmetall, welches in Tröpfchen mit dem Dampf mitgerissen wurde, aufzufangen. Dies gelingt durch den Einsatz eines herkömmlichen Adsorptionsmittels wie Aktivkohle, Silicate oder Aluminiumoxide, welche als Festbett eingesetzt werden. Die Adsorption wird bei einer Temperatur zwischen 30 und 140°C und Raumbelastungen von 0,01 bis 5 1/h durchgeführt.

Das nun vollständig von Katalysatorfracht gereinigte Kopfprodukt 11 wird nun einer Hydrierung 14 zugeführt. Die Hydrierung erfolgt in zwei hintereinander geschalteten Hydrierreaktoren. Der erste Reaktor wird in Schlaufenfahrweise betrieben, der zweite im geraden Durchlauf. Die Hydrierung erfolgt in der Flüssigphase in einem Temperaturbereich von 120 bis 220°C, bevorzugt bei einer Temperatur von 160°C und zwar adiabatisch. Der Druck beträgt 1.5 bis 30 MPa. Die Hydrierung erfolgt in einem heterogenen Festbettkatalysator wie zum Beispiel Kupfer-, Kobalt-, Kupfer/Nickel-, Kupfer/Chrom-, Kupfer/Chrom/Nickel-, Zink/Chrom-, Nickel/Molybdän-Katalysatoren, die gegebenenfalls noch weitere Elemente aufweisen können. Details zur Ausgestaltung einer geeigneten Hydrierung sind in EP0987240B1 und EP0987241B1, sowie in DE102008007080A1 beschrieben.

Das aus der Hydrierung 14 abgezogene Hydrierungsgemisch 15 umfasst nun im Wesentlichen Alkohol, Alkane (aus nicht umgesetzten Olefinen) sowie hydrierte Leichtsieder und Hochsieder. Das Hydrierungsgemisch 15 wird sodann einer thermischen Aufarbeitung 16 zugeführt und dort in eine alkoholreiche Fraktion 17, eine Leichtsiederfraktion 18 und einer Hochsiederfraktion 19 aufgeteilt. Die alkoholreiche Fraktion 17 stellt dabei das eigentliche Wertprodukt des erfindungsgemäßen Verfahrens dar. Die Leichtsieder 18 und Hochsieder 19 stellen Nebenprodukte dar, die sich für untergeordnete Zwecke vermarkten lassen. Die thermische Aufarbeitung 16 der drei Fraktionen 17, 18 und 19 aus dem Hydrierungsgemisch 15 wird anhand der Figur 3 noch näher erläutert werden.

Falls der Umsatz der Alkene in der Hydroformylierung nicht vollständig ist, werden sich die im Kopfprodukt 11 der thermischen Trenneinheit 10 eine größere Menge nicht umgesetzte Alkene finden. Damit diese nicht in der Hydrierung 14 verloren gehen, kann die Hydrierung auch hinter der thermischen Aufarbeitung angeordnet werden, und zwar einmal für die alkoholreiche Fraktion 17 (die in diesem Fall eher aldehydreich ist) und einmal für die Hochsiederfraktion 19. Die Leichtsiederfraktion 18 enthält dann die nicht umgesetzten Alkene und kann in den Hydroformylierungsreaktor 1 zurück geführt werden (nicht in dieser Figur dargestellt). Das Kopfprodukt 11 wird bei einer Alkenrückführung somit nicht vollständig, sondern nur teilweise der Hydrierung zugeführt, die Alkene werden zuvor abgetrennt und zurückgeführt.

Wie bereits erwähnt, finden sich im Sumpfprodukt 12 der thermischen Trenneinheit 10 im Wesentlichen die Hochsieder, geringere Mengen Aldehyd und Katalysator. Der Massenstrom des Sumpfproduktes 12 ist deutlich kleiner als der des Kopfproduktes. Beträgt der Produktstrom 30 Tonnen pro Stunde und wird die Maßgabe eingehalten, dass 90 % der eingetragenen Masse die thermische Trenneinheit 10 über Kopf verlässt, beträgt der Massenstrom des Sumpfproduktes 12 lediglich 3 t pro Stunde, also 1/9 des Kopfproduktes.

Das Sumpfprodukt 12 wird nun einer zweiten Membrantrenneinheit 20 aufgegeben. Dort wird das Sumpfprodukt 12 aufgetrennt in ein Permeat 21 und ein Retentat 22, wobei das Katalysatorsystem im Retentat 22 angereichert wird, da die zweite Membrantrenneinheit 20 das Katalysatorsystem bevorzugt zurückhält. Dank des geringen Massenstroms, den die zweite Membrantrenneinheit 20 im Vergleich zur ersten Membrantrenneinheit 6 aufzutrennen hat, ist es möglich, den im Sumpfprodukt 12 enthaltenen Katalysator nahezu vollständig zurückzuhalten und im Retentat 22 anzureichern. Dies gelingt insbesondere dann, wenn ein Membranmaterial gewählt wird, welches im besonderen Maße für Hochsieder durchlässig ist und folglich die Hochsieder im Permeat 21 anreichert. Retentat 22 besteht dann im Wesentlichen aus Aldehyd und Katalysator.

Die Trennung in der zweiten Membrantrenneinheit 20 erfolgt bei einer Temperatur zwischen 20 und 160°C, bevorzugt zwischen 25 und 90°C und besonders bevorzugt zwischen 30 und 60°C. Die transmembrane Druckdifferenz liegt zwischen 1 bis 10 MPa, bevorzugt zwischen 1.5 und 5 MPa. Besonders bevorzugt wird die Membran bei etwa 2.5 bis 3.5 MPa Transmembrandruck betrieben. Die bevorzugt eingesetzte Membranmodulbauweise ist das Spiralwickelelement.

Die erste und die zweite Membraneinheit können mit denselben oder unterschiedlichen Membranmaterialien arbeiten. Für die betrachteten Beispiele wurden aber immer beide Membraneinheiten mit dem gleichen Membranmaterial ausgelegt.

Ganz besonders bevorzugt werden Membranen aus endständig oder seitenständig organomodifiziertem Siloxanen eingesetzt.

Die zweite Membrantrenneinheit 20 wird als mehrstufige Verstärkungskaskade ausgeführt. Für das Verständnis der Funktion der zweiten Membrantrenneinheit 20 genügt die Annahme, dass es sich um eine einfache Membran handelt.

Das von der zweiten Membrantrenneinheit 20 abgezogene Retentat 22 wird in einem Wärmetauscher 23 auf etwa 40 bis 50°C abgekühlt und sodann mit dem ebenfalls abgekühlten Hydroformylierungsaustrag 4 vermischt und in die erste Membrantrenneinheit 6 zurückgeführt. Das Zurückführen des sekundären Rezyklats (Retentat 22) in die erste Membrantrenneinheit 6 hinter dem Hydroformylierungsreaktor 1 eröffnet den entscheidenden Vorteil, dass regelungstechnische Interferenzen zwischen der Regelung der zweiten Membrantrenneinheit 20 und dem Hydroformylierungsreaktor 1 reduziert werden. Auch wird verhindert, dass unnötigerweise Aldehyd mit dem Retentat 22 in die Reaktion zurückgefahren wird und deren Ausbeute schmälert. Die über den sekundären Rücklauf 22 zurückgeführten Katalysatorbestandteile werden größtenteils von der ersten Membrantrenneinheit 6 wieder abgeschöpft und über den primären Reaktorrücklauf 8 in den Reaktor 1 zurückgeführt.

Das weitestgehend aus Hochsiedern und Rest-Aldehyd bestehende Permeat 21 der zweiten Membrantrenneinheit 20 wird durch einen zweiten Adsorber 24 geleitet, um Restmengen von Katalysator aufzunehmen und zu sichern. Zur Abscheidung von edlen Katalysatormetallen aus dem flüssigen Permeat 21 wird die Adsorption bei einer Temperatur von 30 bis 140°C und Raumbelastungen von 0,01 bis 5 1/h durchgeführt. Bevorzugt wird das Adsorptionsmittel als Festbett eingesetzt.

Als Adsorptionsmittel können insbesondere Aktivkohleoberflächenreiche Polykieselsäuren wie Silicagele (Kiesel-Xerogele), hochdisperse Kieselsäure, oberflächenreiche Aluminiumoxide und Aluminiumoxidhydrate als auch gebrauchte oder neue (Hydrier-)Katalysatoren eingesetzt werden. Als besonders vorteilhafte Adsorbentien haben sich chemisch modifizierte Silica-Materialien erwiesen, wie sie in WO 2006013060 A1 offenbart sind. Derartige Adsorptionsmittel sind unter der Artikelbezeichnung Mercaptoalkyl-modified Silica, Type Rh H3, Batch No. 09-S26-001 der Firma PhosphonicS Ltd, 114 Milton Park, Abingdon, OXON, OX14 4SA, United Kingdom erhältlich.

Das nunmehr vollständig von Katalysatorrückständen adsorptiv gereinigte Permeat 21 wird nun gemeinsam mit dem ebenfalls adsorptiv gereinigten Kopfprodukt 11 der Hydrierung 14 zugeführt. Alternativ wäre es denkbar, Kopfprodukt 11 und Permeat 21 anstelle einer gemeinsamen Hydrierung 14 getrennten Hydrierungsreaktionen zuzuführen (nicht dargestellt).

Es gibt verschiedene Einflussfaktoren auf die Wirtschaftlichkeit des Verfahrens. Zum einen sollte der Rhodiumeinsatzfaktor (entspricht dem Verlust von Rh) möglichst niedrig sein, zum anderen sollten die Investitionskosten, die unter anderem von der benötigten Membranfläche abhängen, nicht zu hoch sein.

Bei den betrachteten Varianten beträgt das Gesamtvolumen des Reaktors beträgt 67 $m^3$. Aspekte wie Wärmeübergang oder geometrische Gestaltung des Reaktors wurden bei der Modellierung nicht berücksichtigt. Der Zulauf an Dibuten beträgt 20 t/hr, so dass bei 8500 Betriebsstunden im Jahr eine Ausbeute von 93 % zum Produkt Nonanal benötigt wird, um den Weltproduktionsmaßstab von 200 kt/a zu erreichen. In allen Membranstufen eine einheitliche Membran mit einem definierten Rückhalt-Permeabilität-Wertepaar eingesetzt. Die für die simulierten Fälle verwendeten Membranwerte (Rückhalt und Permeabilität) entsprechen den in Tabelle 2 zusammengefassten Werten.

Die Simulation gibt zwei bedeutsame Werte aus, die in der folgenden Tabelle 3 dargestellt sind: Dies ist zum einen die aus der Membranpermeabilität resultierende gesamte Membranfläche, die benötigt wird um die benötigte Menge Permeat aus den Membrananlagen zu entnehmen. Angegeben wird die Gesamtfläche der Nanofiltrationseinheiten 6 und 20. Zum anderen ist dies der im Permeat 21 der zweiten Nanofiltrationsstufe enthaltene Massenanteil an Rhodium. Der Permeatstrom 21 beträgt 365 kg/h und ist für alle betrachteten Membranen konstant gehalten worden. Zu diesem Zweck wurde die Membranfläche entsprechend den Permeabilitäten variiert. Mithilfe dieser Werte kann in einer einfachen Betrachtung nachvollzogen werden, welche Membran unter den gegebenen Bedingungen die bevorzugte wäre. Um dies zu verdeutlichen, werden folgende Annahmen getroffen:

• Das Rhodium das die Anlage mit dem Permeatstrom 21 verlässt wird durch den Adsorber 24 geleitet. 81 % des Rhodiums das auf den Adsorber geleitet wird, kann zurückgewonnen werden, Es müssen also nur 19% des im Permeat 21 enthaltenen Rhodiums zu Marktpreisen nachgekauft werden.
• Die Marktpreise für Rhodium werden wie in Beispiel 1 in vier Fällen zu 25, 50, 75 und 100 Euro/g angenommen.
• Für die Membran wird ein Preis von 600 Euro pro Quadratmeter veranschlagt
• Die Investitionskosten für die Membraninstallation bleiben zunächst unberücksichtigt. Unter diesen Annahmen kann eine einfache Betrachtung der Wirtschaftlichkeit durchgeführt werden. Hierzu werden die Werte aus Tabelle 3 mit den oben angegebenen Annahmen zu einem Herstellkostenanteil in Euro pro metrische Tonne Produkt umgerechnet, der direkt von den Rückhalt- und Permeabilitätseigenschaften der Membran abhängt.

Tabelle 3: Membranflächen und Rh-Konzentrationen im Permeat

| Bez. | Membranfläche | $w(Rh)_{Permeat}$ | Herstellkostenanteil [EUR/t Produkt] | | | |
|---|---|---|---|---|---|---|
| | [$m^2$] | [mg/kg] | Rh @ 25 EUR/g | Rh @ 50 EUR/g | Rh @ 75 EUR/g | Rh @ 100 EUR/g |
| A | 3139.2 | 7.1 | 5 | 6 | 8 | 10 |
| B | 9020.0 | 0.4 | 14 | 14 | 14 | 14 |
| C | 6765.0 | 1.3 | 10 | 10 | 10 | 11 |
| D | 9020.0 | 18.4 | 15 | 17 | 18 | 19 |
| E | 1018.8 | 110.3 | 10 | 17 | 26 | 34 |
| F | 5466.7 | 1.4 | 8 | 8 | 9 | 9 |
| G | 2401.1 | 7.0 | 4 | 5 | 5 | 6 |
| H | 2857.4 | 5.9 | 4 | 5 | 6 | 6 |
| I | 1819.8 | 20.0 | 5 | 6 | 9 | 12 |
| J | 3276.0 | 4.5 | 5 | 6 | 6 | 6 |
| K | 1745.8 | 14.6 | 4 | 5 | 6 | 7 |
| L | 2048.4 | 6.7 | 4 | 4 | 5 | 5 |
| M | 956.2 | 40.9 | 4 | 7 | 10 | 13 |
| N | 1380.6 | 20.2 | 4 | 5 | 7 | 8 |
| O | 2505.6 | 4.2 | 4 | 4 | 5 | 5 |
| P | 1573.3 | 7.4 | 3 | 3 | 4 | 5 |

**Beispiel 4** - **Ermittlung des MPI für eine Ru-katalysierte Hydrierung**

Beispiel 4 beschreibt die Vorgehensweise zur Bestimmung des Membran-Leistungs-indikators MPI. Die zu testende Membran wird in einem Querstromfiltrationsexperiment untersucht. Versuchsaufbau und prinzipielles Vorgehen entsprechen der Beschreibung in Beispiel 2. Als Ruthenium-katalysierte Prozesse kommen beispielsweise die Hydroformylierung von Olefinen oder auch die Hydrierung von Carbonyl- und/oder Carboxylverbindungen und ihren Derivaten (Ketone, Aldehyde, Carbonsäuren, Carbonsäureester, Lactone, Amide, Lactame) in Betracht.

In Beispiel 4 werden ausgewählte Membranen aus Beispiel 1 in einem Reaktionssystem enthaltend Isononanol (Wertprodukt), Isononanal (Zwischenprodukt), hochsiedende Nebenprodukte, sowie einen homogenen Hydrierkatalysator eingesetzt. Der Hydrierkatalysator kann als definierter Komplex vorliegen, wahrscheinlicher ist aber eine Mischung verschiedener metallorganischer Komplex-Spezies, sowie deren Abbauprodukte. Der Katalysator bildet sich entweder

in situ aus einem Ru-Metallprecursor (z.B. Ruthenium(III)-acetylacetonat [Ru(acac)$_3$]) und einem Phosphinliganden (z.B. Triphos = 1,1,1-Tris-((diphenylphosphino)methyl)ethan, Tribuphos = 1,1,1-Tris-((di-ortho-(tert.-butyl)phenylphosphino)methyl)ethan) oder aus einem präformierten Ru-Phosphinkomplex (z.B. [(Triphos)Ru(TMM)], mit TMM = Trimethylenmethan).

Die Hydrierung wurde in dem begasten Rührkessel des Versuchsstandes aus Figur 3 durchgeführt. Die Reaktionstemperatur betrug 140°C und es wurde ein Wasserstoffdruck von 50 bar eingestellt. Die Rutheniumkonzentration wurde auf 75 - 100 Milligramm Ruthenium pro Kilogramm Produktgemisch eingestellt, der Ligand Triphos wurde in leichtem Überschuss eingesetzt (1,1 mol/mol Ru). Das Produktgemisch mit o. a. Zusammensetzung verließ den Reaktor und wurde in der Membrantrenneinheit aufgearbeitet. Durch Messungen und Probennahme aus den verschiedenen Strömen (vgl. Beispiel 2) wurden Permeabilität und Ru-Rückhalt der Membran bestimmt. Die Werte sind in Tabelle 4 angegeben und wurden gemäß der beschriebenen Formel in einen MPI umgerechnet. Hierzu wurden die in der Tabelle angegebenen Marktpreise für Ruthenium eingesetzt.

Tabelle 4: Permeabilität und Ru-Rückhalt der Membranen

| Bez. | Ru-Rückhalt | Permeabilität | MPI | | |
|---|---|---|---|---|---|
| | [%] | [kg/(m2 h bar)] | Ru @ 1 EUR/g | Ru @ 5 EUR/g | Ru @ 20 EUR/g |
| A | 94,0 | 0,28 | 0,32 | 0,31 | 0,26 |
| B | 99,0 | 0,11 | 0,05 | 0,04 | 0,04 |
| D | 91,2 | 0,10 | 0,00 | 0,00 | 0,00 |
| F | 97,3 | 0,16 | 0,19 | 0,18 | 0,17 |
| G | 93,8 | 0,37 | 0,36 | 0,35 | 0,31 |
| H | 94,1 | 0,31 | 0,33 | 0,32 | 0,28 |
| L | 94,0 | 0,44 | 0,38 | 0,37 | 0,34 |
| M | 87,6 | 0,94 | 0,44 | 0,42 | 0,34 |
| N | 92,3 | 0,63 | 0,42 | 0,41 | 0,36 |
| P | 93,2 | 0,56 | 0,41 | 0,40 | 0,36 |

**Beispiel 5** - **Prozess zur Hydrierung von Carbonyl- und Carboxygruppen**

Beispiel 5 beschreibt einen Hydrierprozess für Carbonyl- und Carboxylgruppen. Die Beschriebung ist wie üblich vereinfacht, selbstverständliche, nicht erfindungsrelevante Komponenten, wie Ventile, Pumpen usw. werden nicht dargestellt oder beschrieben.

In Beispiel 5 soll Isononanal (INAL) zu Isononanol (Isononylalkohol, INA) hydriert werden. Die Kapazität der Anlage beträgt 50000 Tonnen pro Jahr bei einer Verfügbarkeit der Anlage von 8000 Stunden pro Jahr. INAL und Wasserstoff werden einem Reaktor (z.B. ein Blasensäulenreaktor oder oder ein begaster Rührkesselreaktor) zugeführt. In dem Reaktor findet unter Reaktionsbedingungen (Temperatur 150 °C und Druck 50 bar) die Umwandlung des INAL zu INA und Neben- und/oder Folgeprodukten statt. Den Reaktor verlassen überschüssiger Wasserstoff, sowie gasförmige Nebenprodukte oder Inertstoffe und werden als Abgas abgeführt. Der flüssige Produktaustrag wird in eine Nanofiltrationseinheit geleitet wo eine Abtrennung des Katalysators stattfindet. Die Einheit ist zweistufig verschaltet um den Rückhalt zu erhöhen, die benötigte Membranfläche ergibt sich aus der Permeabilität der Membranen und ist in Tabelle 5 dargestellt. An Katalysator abgereichertes Permeat kann dann weiteren Aufarbeitungsschritten zugeführt werden (bspw. Destillation). Der Rutheniummassenanteil w(Ru) im Permeat ist in Tabelle 5 angegeben. Katalysatorreiches Retentat wird als Katalysatorkreislauf zurück in den Reaktor geführt. Die anteiligen Herstellkosten wurden unter folgenden Annahmen abgeschätzt:

Das Gesamte Ruthenium, das die Anlage über das Permeat der Nanofiltration verlässt muss zu Marktpreisen nachgekauft werden. Drei angenommene Marktpreise wurden in die Berechnung einbezogen und sind in Tabelle 5 angegeben.

Die Membranen haben alle einen Marktpreis von 1000 EUR pro Quadratmeter und eine Standzeit von 2 Jahren, so dass jedes Jahr die Hälfte der benötigten Membranfläche ersetzt werden muss. Die Erstbestückung zählt zu den Investitionskosten und wird in der Berechnung nicht berücksichtigt.

Tabelle 5: Ru-Massenanteil, Membranfläche und anteilige Herstellkosten

| Bez. | w(Ru) | Membranfläche | Herstellkostenanteil [EUR/t Produkt] | | |
|---|---|---|---|---|---|
| | mg/kg | m2 | Ru @ 1 EUR/g | Ru @ 5 EUR/g | Ru @ 20 EUR/g |
| A | 0.34 | 1488.10 | 15.22 | 16.60 | 21.76 |
| B | 0.01 | 3787.88 | 37.89 | 37.93 | 38.07 |
| D | 0.74 | 4275.79 | 43.50 | 46.46 | 57.55 |
| F | 0.07 | 2591.39 | 25.99 | 26.28 | 27.36 |
| G | 0.37 | 1138.19 | 11.75 | 13.22 | 18.72 |
| H | 0.33 | 1354.53 | 13.88 | 15.21 | 20.19 |
| L | 0.34 | 946.97 | 9.81 | 11.18 | 16.32 |
| M | 1.47 | 443.26 | 5.90 | 11.78 | 33.80 |
| N | 0.57 | 661.38 | 7.18 | 9.45 | 17.94 |
| P | 0.44 | 745.82 | 7.90 | 9.67 | 16.29 |

**Fazit**

Die Beispiele verdeutlichen, dass ein im Stand der Technik vorherrschendes Vorurteil von den erfindungsgemäßen Ausführungen widerlegt wird. So wird zum Beispiel in DE102009047351A1 ausdrücklich darauf hingewiesen, dass von einer hinreichenden Trennleistung einer Membran erst ausgegangen werden kann, wenn mindestens 95 % der zurückzuhaltenden Komponente auch tatsächlich zurückgehalten werden. Die obigen Beispiele belegen hingegen, dass eine solch reduzierte Betrachtung der komplexen Thematik der Organophilen Nanofiltration nicht gerecht wird. Beispielsweise würde der Fachmann aufgrund des beschrieben Vorurteils von den aktuell kommerziell verfügbaren Membranen (Membranen A bis D), die Membranen B oder C auswählen, da nur diese eine Katalysatorrückhalt von mehr als 95 % aufweisen. In einem Verfahren, dass nach diesen Grundsätzen ausgelegt würde, würde dies zu einem Herstellkostenanteil (durch Metall-Verlust und Membrankosten) 10 bis 14 Euro pro Tonne Produkt führen (unter den in Beispiel 3 genannten Rahmenbedingungen). In einem nach dem erfindungsgemäßen Vorgehen entwickeltes Verfahren würde schon bei den kommerziell verfügbaren Membranen die Membran A auswählen, da diese zwar einen Rückhalt unter 95 % aufweist, der MPI aber zumindest bei niedrigen Metallpreisen bei vorteilhaften 0,35 liegt. Diese Membran würde in einem Beispielprozess nur zu Kosten in der Größenordnung von 5 Euro pro Tonne Produkt führen. Die nach dem Stand der Technik gewählten Membranen weisen hingegen MPI-Werte deutlich unter 0,35 auf und würden, zurecht, in einem erfindungsgemäß entwickelten Verfahren nicht verwendet. Weiterhin zeigen die Beispiele, dass es für ein nach erfindungsgemäßem Vorgehen entwickeltes erfindungsgemäßes Verfahren vorteilhaft ist, Membranen mit einer trennaktiven Schicht aus Siliconacrylaten (Membranen E bis P) zu verwenden.

Zunächst wird deutlich, dass durch Verwendung weniger Komponenten eine sehr breite Palette an Membranen gefertigt werden kann. Durch Anpassung der Beschichtungsbedingungen (Abrollgeschwindigkeit der Unterstruktur und Polymerkonzentration im Tauchbad) und/oder durch Variation der Zusammensetzung der Siliconacrylatmischung können die Membranparameter Rückhalt und Permeabilität in einem weiten Feld eingestellt werden. So variiert bei den erfindungsgemäß hergestellten Membranen beispielsweise der Rückhalt von ca. 75% bis über 97% und die Permeabilität von 0,5 kg/($m^2$ h bar) bis über 2,8 5 kg/($m^2$ h bar). Diese Variabilität ermöglicht die erfindungsgemäße Auslegung eines Verfahrens. Es wird deutlich, dass fast alle Siliconacrylatmembranen einen MPI über 0,35 aufweisen, und viele sogar von 0,45 oder höher. Die Verwendung solcher Membranen ist besonders vorteilhaft, wie aus den angegebenen, beispielhaften Herstellkostenanteilen abgeleitet werden kann. Bei der Beurteilung ist es wichtig zu berücksichtigen, dass die MPI-Werte in Tabelle 2 nur spaltenweise (also bei gleichem angenommenem Metallpreis) verglichen werden sollten. So kann für den betrachteten Fall die Ideale Membran ausgewählt werden.

Da sich die Rahmenbedingungen beim langjährigen Betrieb eines Prozesses verändern können (insbesondere die Edelmetallpreise, die einen signifikanten Anteil der Katalysatorkosten eines Prozesses ausmachen, unterliegen oft deutlichen mittel bis langfristig relevanten Schwankungen), ist eine regelmäßige Neubewertung eines laufenden Prozesses mit Hilfe des MPI sinnvoll. Da alle Polymermembranen einer gewissen Alterung unterliegen müssen sie in regelmäßigen Abständen ausgetauscht werden. In Abhängigkeit von den Stoffsystemen (Membran und zu trennende Stoffströme) können Standzeiten eines Membranmoduls von wenigen Monaten bis zu einigen Jahren möglich sein. In der Praxis sind Standzeiten von 6 bis 24 Monaten nicht unüblich. Der Vorteil der laufenden Bewertung mittels des MPI in Verbindung mit der Verwendung der flexiblen Siliconacrylat-Technologieplattform ermöglicht es nun vor jedem Austausch der Membranmodule eine Bewertung vorzunehmen und das zum jeweiligen Zeitpunkt optimale Membranmaterial für die Wiederbestückung auszuwählen. Da mit geringen Anpassungen in der Herstellung des Membranmaterials große Effekte erzielt werden können, sind Siliconacrylat-Membranen für eine derartige Prozessführung besonders gut geeignet.

Auch bei den Membranen E bis P zeigt sich, dass eine Bewertung alleine auf Basis des Rückhalts nicht zielführend ist, weil nicht immer die Membranen mit dem höchsten Rückhalt auch den besten (höchsten) MPI aufweisen. Aus Tabelle 3 ist aber sofort ersichtlich, dass die Membranen mit einem geringeren MPI in der Regel zu höheren Herstellkosten führen.

## Patentansprüche

1. Verfahren zur Abtrennung eines Homogenkatalysators oder zumindest von Bestandteilen oder Abbauprodukten desselben aus einem Reaktionsgemisch mit Hilfe organophiler Nanofiltration, bei dem ein Reaktionsgemisch umfassend ein flüssiges organisches Lösemittel und darin gelöst ein Homogenkatalysator und/oder Bestandteile und/oder Abbauprodukte des Homogenkatalysators auf eine Membran gegeben und daran getrennt wird in ein Retentat und in ein Permeat, wobei der Homogenkatalysator bzw. seine Bestandteile und/oder Abbauprodukte in dem Retentat dergestalt angereichert wird, dass die Konzentration des Homogenkatalysators bzw. seiner Bestandteile und/oder Abbauprodukte in dem Retentat größer ist als in dem Permeat und in dem Reaktionsgemisch, und wobei es sich bei dem Homogenkatalysator um eine metallorganische Komplexverbindung mit einem Metall als Zentralatom handelt, **dadurch gekennzeichnet,**
**dass** die Abtrennung so geregelt wird, dass sie bei einem dimensionslosen Membran-Leistungsindikator MPI von größer als 0.35 erfolgt, wobei der Membran-Leistungsindikator wie folgt definiert ist:

$$MPI := 0.5\ R^{S/7}\ (1+P)^{S/(100*R)} - 1/(10*P)$$

worin R für den dimensionslosen Rückhalt der Membran hinsichtlich des Metalls oder hinsichtlich der anderen Bestandteile und/oder Abbauprodukte des Homogenkatalysators steht und P für die Permeabilität der Membran in kg/(m$^2$*bar*h) steht und worin S für einen angenommenen oder den gegenwärtigen Marktpreis des Metalls bzw. der anderen Bestandteile und/oder Abbauprodukte des Homogenkatalysators in Euro/g steht.

2. Vorrichtung zur Abtrennung von Homogenkatalysator und/oder zumindest von Bestandteilen und/oder Abbauprodukte desselben aus einem Reaktionsgemisch im Wege der organophilen Nanofiltration, mit einer Membran, die einen Zulauf für einen Feed, einen Ablauf für ein Retentat und einen Ablauf für ein Permeat aufweist, **dadurch gekennzeichnet, dass** die Membran so bemessen ist, dass ein dimensionsloser Membran-Leistungsindikator MPI im bestimmungsgemäßen Betrieb der Vorrichtung mindestens 0.35 beträgt, wobei der Membran-Leistungsindikator wie folgt definiert ist:

$$MPI := 0.5\ R^{S/7}\ (1+P)^{S/(100*R)} - 1/(10*P)$$

worin R für den dimensionslosen Rückhalt der Membran hinsichtlich des Metalls oder der anderen Bestandteile und/oder Abbauprodukte des Homogenkatalysators steht und P für die Permeabilität der Membran in kg/(m$^2$*bar*h) steht und worin S für einen angenommenen oder den gegenwärtigen Marktpreis des Metalls bzw. der anderen Bestandteile und/oder Abbauprodukte des Homogenkatalysators in Euro/g steht, und wobei es sich bei dem Homogenkatalysator um eine metallorganische Komplexverbindung mit dem besagten Metall als Zentralatom handelt, und wobei die Vorrichtung eine Regelung aufweist, die dafür eingerichtet ist, zumindest einen Betriebsparameter der Vorrichtung so zu steuern, dass der Ist-Wert des Membran-Leistungsindikators der Vorrichtung auf einen größer oder gleich 0.35 eingestellten Soll-Wert des Membran-Leistungsindikators geregelt wird.

3. Gegenstand nach einem der Ansprüche 1 bis 2, wobei der MPI mindestens 0.5 oder mehr als 0.75 beträgt.

4. Gegenstand nach Anspruch 1 oder 2, wobei die Membran eine trennaktive Schicht aus Siliconacrylat umfasst.

5. Gegenstand nach einem der Ansprüche 1 bis 4, **dadurch gekennzeichnet, dass** das Reaktionsgemisch mittelbar oder unmittelbar aus einer der chemischen Reaktion stammt, die ausgewählt ist aus der Gruppe umfassend die folgenden Reaktionstypen:

Hydroformylierung, Hydrocarboxylierung, Alkoxycarbonylierung, Methoxycarbonylierung, Hydrierung, Hydrocyanierung;
und dass es sich bei dem Homogenkatalysator um eine metallorganische Komplexverbindung handelt, dessen Zentralatom ausgewählt ist aus der Gruppe der umfassend die folgenden Metalle: Cobalt, Rhodium, Iridium, Ruthenium, Palladium;
unter der Maßgabe, dass der ausgewählte Homogenkatalysator die ausgewählte chemische Reaktion dergestalt katalysiert, sodass das Reaktionsgemisch erhalten wird.

## Claims

1. Process for separating a homogeneous catalyst or at least constituents or degradation products thereof from a reaction mixture by means of organophilic nanofiltration, in which a reaction mixture comprising a liquid organic solvent and a homogenous catalyst or constituents and/or degradation products of the homogeneous catalyst dissolved therein is supplied to a membrane and separated thereon into a retentate and a permeate, where the homogeneous catalyst or the constituents and/or degradation products thereof accumulate in the retentate in such a way that the concentration of the homogeneous catalyst or the constituents and/or degradation products thereof is greater in the retentate than in the permeate and in the reaction mixture, and the homogeneous catalyst is a metal-organic complex having a metal as central atom, **characterized in that** the separation is regulated so that it occurs at a dimensionless membrane performance indicator MPI of greater than 0.35, where the membrane performance indicator is defined as follows:

$$MPI = 0.5\ R^{S/7}\ (1+P)^{S/(100*R)} - 1/(10*P)$$

where R is the dimensionless retention of the membrane in respect of the metal or in respect of the other constituents and/or degradation products of the homogeneous catalyst and P is the permeability of the membrane in kg/ ($m^2$*bar*h) and S is an assumed market price or the current market price of the metal or of the other constituents and/or degradation products of the homogeneous catalysts in euro/g.

2. Apparatus for separating homogeneous catalysts and/or at least constituents and/or degradation products thereof from a reaction mixture by means of organophilic nanofiltration using a membrane which has an inlet for a feed, an outlet for a retentate and an outlet for a permeate, **characterized in that** the membrane is dimensioned such that a dimensionless membrane performance indicator MPI in correct operation of the apparatus is at least 0.35, where the membrane performance indicator is defined as follows:

$$MPI = 0.5\ R^{S/7}\ (1+P)^{S/(100*R)} - 1/(10*P)$$

where R is the dimensionless retention of the membrane in respect of the metal or in respect of the other constituents and/or degradation products of the homogeneous catalyst and P is the permeability of the membrane in kg/ ($m^2$*bar*h) and S is an assumed market price or the current market price of the metal or of the other constituents and/or degradation products of the homogeneous catalyst in euro/g and the homogeneous catalyst is a metal-organic complex having the said metal as central atom and the apparatus has a regulating facility which is equipped for controlling at least one operating parameter of the apparatus in such a way that the actual value of the membrane performance indicator of the apparatus is regulated to an intended value of the membrane performance indicator which is set to greater than or equal to 0.35.

3. Subject matter according to either Claim 1 or 2, wherein the MPI is at least 0.5 or more than 0.75.

4. Subject matter according to Claim 1 or 2, wherein the membrane comprises a separation-active layer composed of silicone acrylate.

5. Subject matter according to any of Claims 1 to 4, **characterized in that** the reaction mixture originates indirectly or directly from a chemical reaction selected from the group consisting of the following types of reaction:

hydroformylation, hydrocarboxylation, alkoxy-carbonylation, methoxycarbonylation, hydrogenation, hydrocyanation;
and **in that** the homogeneous catalyst is a metal-organic complex whose central atom is selected from the group consisting of the following metals: cobalt, rhodium, iridium, ruthenium, palladium;
with the proviso that the selected homogeneous catalyst catalyses the selected chemical reaction so as to obtain the reaction mixture.

## Revendications

1. Procédé de séparation d'un catalyseur homogène ou au moins de constituants ou de produits de décomposition de celui-ci d'un mélange réactionnel à l'aide d'une nanofiltration organophile, selon lequel un mélange réactionnel comprenant un solvant organique liquide et un catalyseur homogène dissous dans celui-ci et/ou des constituants et/ou des produits de décomposition du catalyseur homogène est introduit sur une membrane et y est séparé en un rétentat et en un perméat, le catalyseur homogène ou ses constituants et/ou produits de décomposition s'accumulant dans le rétentat, de telle sorte que la concentration du catalyseur homogène ou de ses constituants et/ou produits de décomposition dans le rétentat soit supérieure à celle dans le perméat et dans le mélange réactionnel, et le catalyseur homogène consistant en un composé complexe métallo-organique comprenant un métal en tant qu'atome central, **caractérisé en ce que** la séparation est ajustée de telle sorte qu'elle ait lieu à un indicateur de puissance de membrane MPI sans dimension supérieur à 0,35, l'indicateur de puissance de membrane étant défini par :

$$MPI = 0,5\ R^{S/7}\ (1+P)^{S/(100*R)} - 1/(10*P)$$

R représentant la rétention de la membrane sans dimension au regard du métal ou au regard des autres constituants et/ou produits de décomposition du catalyseur homogène, et P représentant la perméabilité de la membrane en kg/($m^2$*bar*h) et S représentant un prix du marché supposé ou le prix du marché actuel du métal ou des autres constituants et/ou produits de décomposition du catalyseur homogène en Euro/g.

2. Dispositif pour la séparation d'un catalyseur homogène et/ou au moins de constituants et/ou produits de décomposition de celui-ci d'un mélange réactionnel par nanofiltration organophile, comprenant une membrane, qui comprend une entrée pour une alimentation, une sortie pour un rétentat et une sortie pour un perméat, **caractérisé en ce que** la membrane est dimensionnée de telle sorte qu'un indicateur de puissance de membrane MPI sans dimension en mode normal du dispositif soit d'au moins 0,35, l'indicateur de puissance de membrane étant défini par :

$$MPI = 0,5\ R^{S/7}\ (1+P)^{S/(100*R)} - 1/(10*P)$$

R représentant la rétention de la membrane sans dimension au regard du métal ou au regard des autres constituants et/ou produits de décomposition du catalyseur homogène, et P représentant la perméabilité de la membrane en kg/($m^2$*bar*h) et S représentant un prix du marché supposé ou le prix du marché actuel du métal ou des autres constituants et/ou produits de décomposition du catalyseur homogène en Euro/g, et le catalyseur homogène consistant en un composé complexe métallo-organique comprenant ledit métal en tant qu'atome central, et le dispositif comprenant un réglage qui est conçu pour ajuster au moins un paramètre d'exploitation du dispositif de telle sorte que la valeur réelle de l'indicateur de puissance de membrane du dispositif soit ajustée à une valeur de consigne supérieure ou égale à 0,35 de l'indicateur de puissance de membrane.

3. Article selon l'une quelconque des revendications 1 à 2, dans lequel le MPI est d'au moins 0,5 ou supérieur à 0,75.

4. Article selon la revendication 1 ou 2, dans lequel la membrane comprend une couche à action de séparation en acrylate de silicone.

5. Article selon l'une quelconque des revendications 1 à 4, **caractérisé en ce que** le mélange réactionnel provient indirectement ou directement d'une réaction chimique qui est choisie dans le groupe comprenant les types de réactions suivants : hydroformylation, hydrocarboxylation, alcoxycarbonylation, méthoxycarbonylation, hydrogénation, hydrocyanation ;
et **en ce que** le catalyseur homogène consiste en un composé complexe métallo-organique dont l'atome central

est choisi dans le groupe comprenant les métaux suivants : cobalt, rhodium, iridium, ruthénium, palladium ;
à condition que le catalyseur homogène choisi catalyse la réaction chimique choisie de telle sorte que le mélange réactionnel soit obtenu.

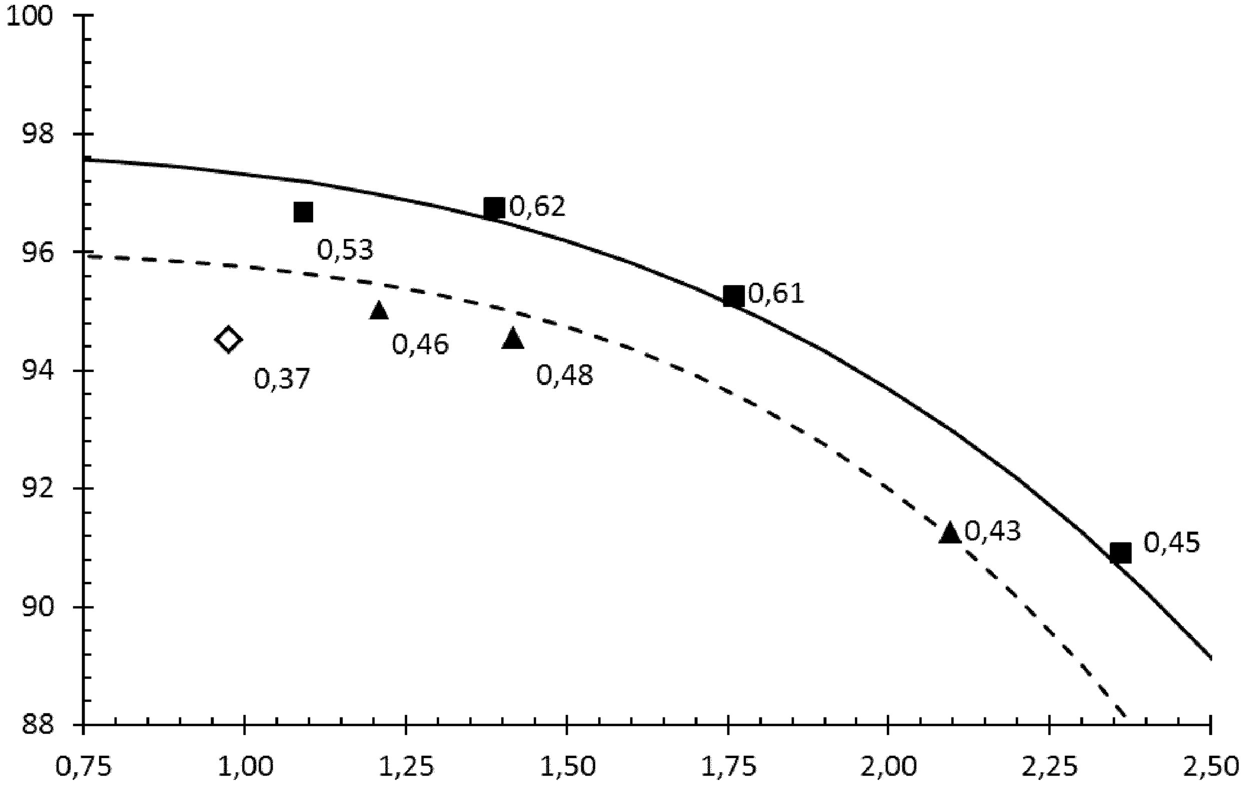
100
98
96
94
92
90
88
0,75
1,00
1,25
1,50
1,75
2,00
2,25
2,50
0,62
0,53
0,61
0,46
0,37
0,48
0,43
0,45

Fig.1

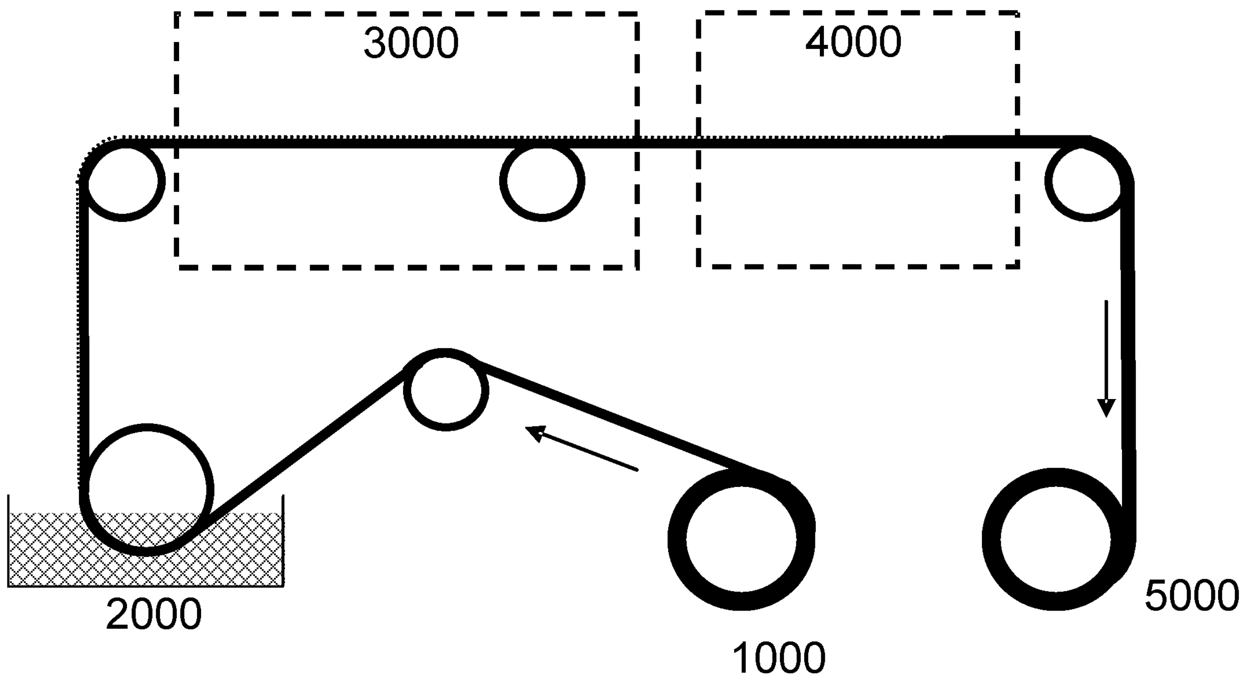


Fig. 2

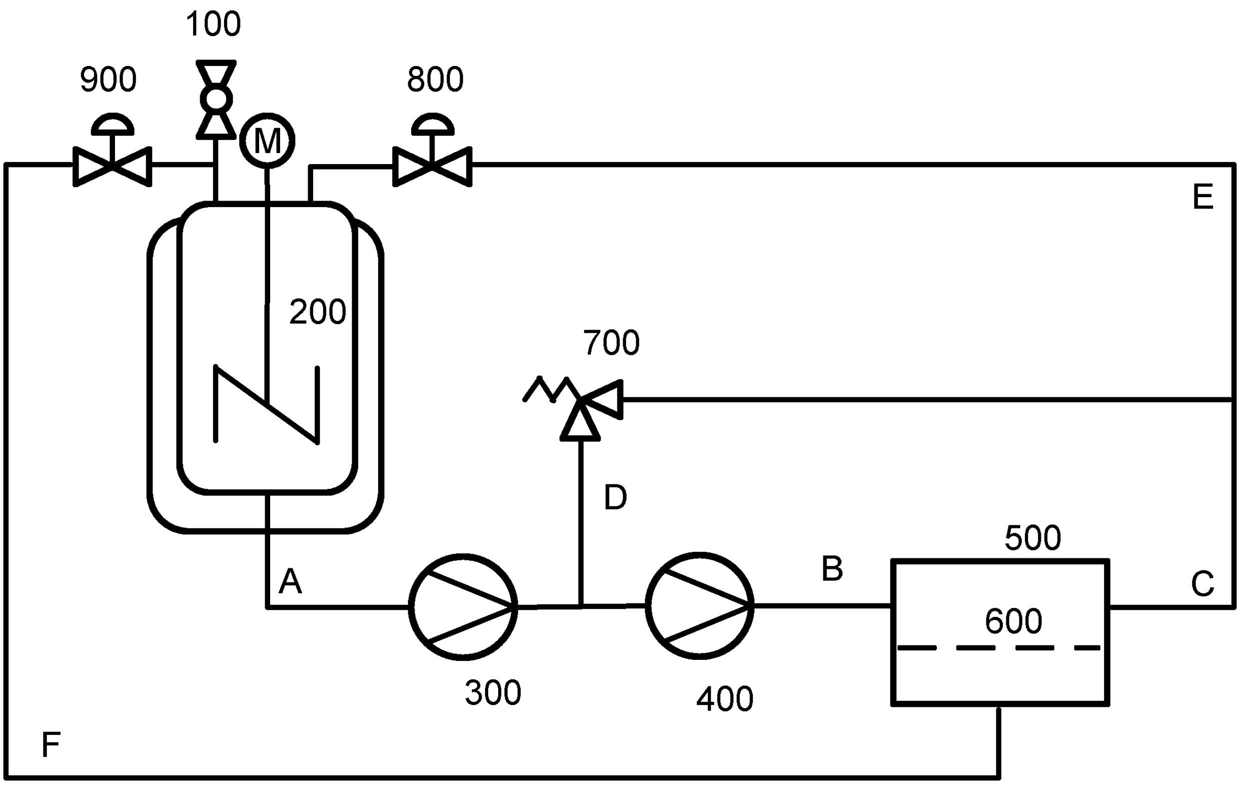


Fig. 3

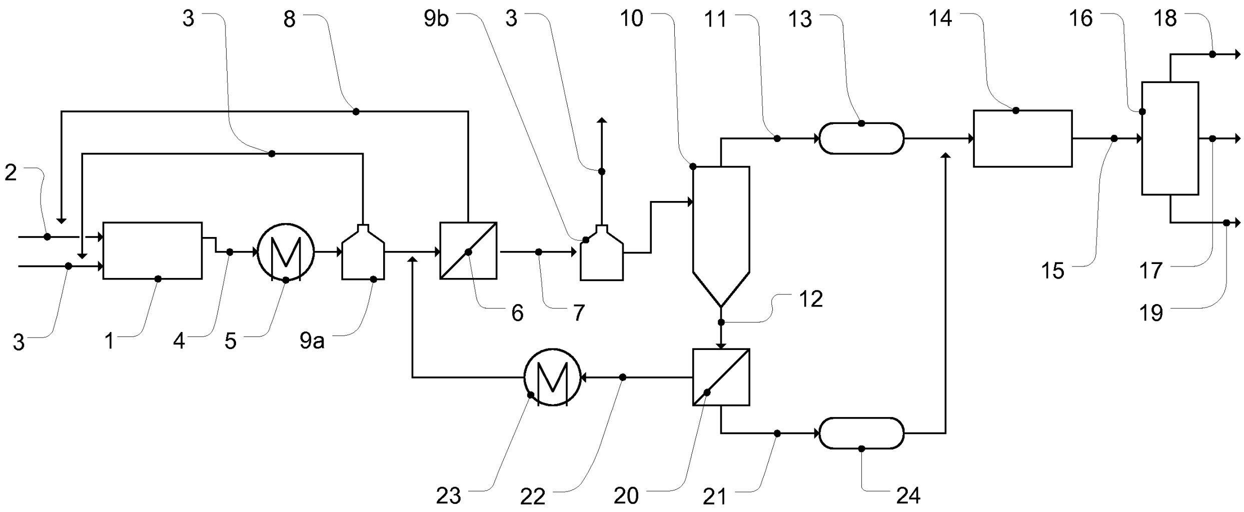


Fig. 4

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1931472 B1
- WO 2014183952 A1
- WO 2011067054 A1
- EP 0330076 A2
- DE 102013203117 A1
- EP 1603663 B1
- EP 0781166 A
- EP 0987240 B1
- EP 0987241 B1
- DE 102008007080 A1
- WO 2006013060 A1
- DE 102009047351 A1


### In der Beschreibung aufgeführte Nicht-Patentliteratur


- Grundlagen der Modul- und Anlagenauslegung. **MELIN ; RAUTENBACH.** Membranverfahren. Springer, 2004
- **PRISKE, M. et al.** Reaction integrated separation of homogeneous catalysts in the hydroformylation of higher olefins by means of organophilic nanofiltration. *Journal of Membrane Science,* 15. September 2010, vol. 360 (1-2), 77-83
- *Chem. Soc. Rev.,* 2008, vol. 37, 365-405
- **MARRIOT J et al.** the optimal design of membrane systems. *Chm. Eng. Sc,* 2003, vol. 58 (22), 4991-5004
- Bubble Columns. **DEEN, N.G. ; MUDDE, R.F. ; KUIPERS, J.A.M. ; ZEHNER, P. ; KRAUME, M.** Ullmann's Encyclopedia of Industrial Chemistry. 15. Januar 2010
- Membranes. **I. CABASSO.** Encyclopedia of Polymer Sience and Technlogy. John Wiley and Sons, 1987